Europäisches Patentamt

**European Patent Office**

**Office européen des brevets**

(11) Numéro de publication: **0 264 150 B1**

(12) **FASCICULE DE BREVET EUROPEEN**

(45) Date de publication de fascicule du brevet: **07.01.93** (51) Int. Cl.⁵: **C12N 15/62**

(21) Numéro de dépôt: **87201873.4**

(22) Date de dépôt: **30.09.87**

(54) **Microorganismes comportant des pili comme protéines porteuses, pili isolés à partir de ces microorganismes, procédé d'excrétion de peptides ou de protéines à l'aide de ces pili et utilisation de ceux-ci.**

(30) Priorité: **13.10.86 FR 8614294**
**26.11.86 FR 8616610**

(43) Date de publication de la demande:
**20.04.88 Bulletin 88/16**

(45) Mention de la délivrance du brevet:
**07.01.93 Bulletin 93/01**

(84) Etats contractants désignés:
**AT BE CH DE ES FR GB GR IT LI LU NL SE**

(56) Documents cités:
**EP-A- 0 060 129**
**EP-A- 0 174 759**
**WO-A-86/02557**
**GB-A- 2 094 314**

**CONFERENCE PAPERS INDEX, CAMBRIDGE SCIENTIFIC ABSTRACTS, 1986, résumé no. 86007853, Washington, US; D. NEWMAN et al.: "Extracellular expression of a linear viral epitope inserted into the K88ab pilus structural subunit"**

(73) Titulaire: **SOLVAY**
**Rue du Prince Albert, 33**
**B-1050 Bruxelles(BE)**

(72) Inventeur: **Thiry, Georges**
**Avenue du Loriot, 22**
**B-1150 Bruxelles(BE)**
Inventeur: **Clippe, André**
**Rue Culot, 33**
**B-7030 Saint-Symphorien(BE)**
Inventeur: **Scarcez, Thierry**
**Rue du Happart, 48**
**B-7990 Sirault(BE)**
Inventeur: **Petre, Jean**
**Rue Charles Lemaire, 26**
**B-1160 Bruxelles(BE)**

(74) Mandataire: **Lechien, Monique et al**
**Solvay Département de la Propriété Industrielle Rue de Ransbeek, 310**
**B-1120 Bruxelles(BE)**

EP 0 264 150 B1

CHEMICAL ABSTRACTS, vol. 96, no. 25, 21 juin 1982, pages 163-164, résumé no. 211670p, Columbus, Ohio, US; F.R. MOOI et al.: "Construction and characterization of mutants impaired in the biosynthesis of the K88ab antigen", & J. BACTERIOL. 1982, 150(2), 512-21

MICROBIOLOGICAL REVIEWS, vol. 46, no. 2, juin 1982, pages 129-161, US; W. GAASTRA et al.: "Host-specific fimbrial adhesins of no-ninvasive enterotoxigenic Escherichia coli strains"

EP 0 264 150 B1

**Description**

La présente invention concerne des microorganismes comportant des pili comme protéines porteuses de matériel peptidique hétérologue ainsi que les pili isolés à partir de tels microorganismes. L'invention concerne également un procédé d'excrétion de peptides ou de protéines utilisant les mécanismes propres des microorganismes pour la production de pili. L'invention concerne aussi l'utilisation des microorganismes et des pili.

Les mécanismes de production des pili d'Escherichia coli et plus particulièrement des sous-unités fimbriales K88 et K99 appelées dans la nouvelle nomenclature respectivement F4 et F5 sont actuellement connus et ont été décrits dans la littérature par notamment :
- B. Oudega, F.R. Mooi et F.K. de Graaf-Antonie van Leeuwenhoek 50, 1984, 569-584
- M.R. Mooi, F.K. de Graaf-Symp. Biotechnol. Res. Neth. 1983
- M. Kehoe, R. Sellwood, R. Shipley and G. Dougan - Nature Vol. 291 du 14 mai 1981
- J. Josephsen, F. Hansen, F.K. de Graaf et W. Gaastra FEMS. Microbiology Letters 25 (1984) 301-306
- D. Newman, B. Hay et Sadowski - Federation Proceedings 1985, 44 (5), p. 1483

Dans ces documents aucune information n'est cependant fournie sur l'utilisation de ces mécanismes pour l'obtention de microorganismes comportant des pili comme protéines porteuses permettant l'expression de peptides ou de protéines hétérologues.

Il est cependant connu par le titre de la conférence (Conference Papers Index, Cambridge Scientific Abstracts 1986 - Résumé n° 86007853) qu'il est possible d'obtenir l'expression extracellulaire d'un épitope viral linéaire inséré dans la sousunité du pilus K88 ab.

Il est également connu par la demande de brevet européen 0 174 759 le principe d'obtention d'une protéine conjuguée comprenant une partie d'une protéine porteuse qui s'agglomère normalement à une protéine immunogénique et une fraction d'un peptide qui contient l'épitope d'un antigène lié à la protéine porteuse. La seule protéine porteuse exemplifiée est celle de la membrane du virus de la mosaïque du tabac et le seul peptide exemplifié est celui du virus de la polio types 1 et 3.

La présente invention fournit les moyens pour obtenir en pratique des microorganismes comportant des pili comme protéines porteuses permettant l'expression de certains peptides ou de certaines protéines hétérologues.

L'invention concerne à cet effet des microorganismes recombinants dont la membrane externe porte des pili dont la composition a été modifiée par au moins deux changements de la séquence protéique de la sous-unité, changements qui résultent de l'insertion dans le gène codant pour la sous-unité d'au moins deux fragments d'ADN codant pour un peptide ou une protéine hétérologue, faisant partie d'une région antigénique du virus de l'Influenza et/ou de l'hemagglutinine du virus de l'Influenza et/ou étant un peptide de l'hormone somatostatine.

La nature des microorganismes concernés par l'invention n'est pas critique en elle-même et dépend uniquement de la possibilité que possèdent certains microorganismes de produire des pili tels que définis ci-avant. Habituellement, on opère avec des bactéries comme microorganismes.

Parmi celles-ci sont mises en oeuvre, de préférence, les bactéries des familles Enterobacteriaceae, Streptococcaceae, Neisseriaceae, Proteeae, Rhizobiaceae, Pseudomonodaceae, Corynebacteriaceae, Mycoplasmataceae, Myxococcaceae, Actinomycetaceae, Bacteroidaceae et, de manière préférée, les bactéries des genres Escherichia, Neisseria, Proteus, Bordetella, Pseudomonas, Salmonella, Klebsiella, Moraxella, Streptococcus, Serratia, Enterobacter, Agrobacterium, Rhizobium, Corynebacterium, Myxococcus, Actinomyces, Caulobacter, Bacteroides, Shigella, Mycoplasma et, de manière particulièrement préférée, les espèces Escherichia coli, Neisseria gonorrhoeae, Proteus mirabilis, Bordetella pertussis, Pseudomonas aeruginosa, Pseudomonas echinoïdes, Streptococcus pneumoniae, Neisseria meningitidis, Klebsiella pneumoniae. Enfin, de bons résultats ont été obtenus avec les souches d'Escherichia coli, en particulier les souches de sérotype K88 et K99.

L'invention concerne également les pili isolés de ces microorganismes par tout moyen physique, chimique ou biologique. Elle concerne également les sous-unités ou pilines qui, par quelque moyen que ce soit, ont été libérées et séparées de la structure polymérique répétitive constituant les pili.

Par pili on entend également les termes tels que fibrilles et, fimbriae. Ce sont des polymères excrétés par les microorganismes et attachés à la membrane externe de ces microorganismes, constitués de la répétition de protéines appelées pilines ou encore sous-unités.

Les sous-unités ou pilines, dont la répétition constitue le pilus, sont des protéines qui ont généralement un poids moléculaire compris entre 5 et 50 kD et qui, lorsqu'elles sont associées sous forme de pili, forment des filaments extracellulaires de 20 à 250 Å de diamètre et pouvant atteindre plusieurs microns de long.

EP 0 264 150 B1

Les pili décrits dans l'invention peuvent être de tous types tels que des pili somatiques.

De préférence on utilise les pili somatiques qui servent à l'adhésion des microorganismes sur un support et de bons résultats ont été obtenus avec les pili K88 d'Escherichia coli qui permettent à cette bactérie de coloniser l'intestin de certains mammifères.

Les pili obtenus selon l'invention ont une composition différente de la composition des pili du microorganisme à l'état sauvage suite à au moins une modification apportée à la séquence protéique de la sous-unité ou piline, de manière préférée suite à au moins deux modifications.

Les modifications de la sous-unité portent de préférence sur les régions non critiques pour l'assemblage et l'excrétion de la sous-unité.

La protéine obtenue après modifications peut être utilisée dans diverses applications. En fonction du ou des piptides introduits dans la sous-unité elle peut notamment convenir comme enzyme, comme constituant nutritif ou encore dans le domaine de la santé animale ou humaine notamment en tant que produits pharmaceutiques à usage vétérinaire ou humain. Dans ce cas, la protéine obtenue peut être administrée sous n'importe quelle forme pharmaceutiquement acceptable telle que notamment la protéine isolée, la protéine liée au pilus isolé ou non du microorganisme; le microorganisme lui-même peut donc être administré.

Les pili sont des molécules fortement antigéniques. Si le peptide introduit est un épitope étranger au pilus, la protéine obtenue selon l'invention peut posséder un caractère antigénique nouveau et être utilisée dans des tests immunologiques tels que pour des diagnostics. Elle peut également posséder un caractère immunogénique et être utilisée comme composant d'un vaccin.

L'invention permet également de rendre immunogéniques des peptides, qui ne le sont que peu ou pas par eux-mêmes, après leur introduction dans la séquence protéique de la piline.

La modification de la séquence protéique de la sous-unité ou piline peut être réalisée par tout moyen connu.

Un moyen est de modifier la séquence d'ADN codant pour la sous-unité du pilus. Ces modifications sont réalisées par ingéniérie génétique. Le gène recombinant est ensuite traduit en protéines (hétérologues) dans un organisme hôte.

Par modification on entend toute modification de l'ADN sauvage, conduisant à une modification de la séquence protéique de la sous-unité.

On entend également le remplacement d'un fragment d'ADN sauvage par un fragment d'ADN étranger. L'ADN étranger peut être d'origine naturelle ou synthétique. Sa séquence en nucléotides et sa phase de lecture dans le gène déterminent la séquence native en acides aminés incorporés dans la sous-unité.

L'invention concerne dès lors également tout matériel génétique permettant l'utilisation de pili comme protéines porteuses.

L'invention concerne en outre les procédés permettant de réaliser des pili en tant que protéines porteuses par incorporation d'ADN hétérologue ainsi que les procédés d'obtention de microorganismes porteurs de tels pili. Plus particulièrement l'invention concerne tout procédé dans lequel le gène recombinant permet d'obtenir des pili dont la structure polymérique est conservée et contenant éventuellement les sous-unités modifiées résultant de l'incorporation de l'ADN hétérologue.

L'invention concerne donc les procédés qui permettent d'obtenir des pili dont la composition a été modifiée par au moins un changement de la séquence protéique de la sous-unité, ce ou ces changements résultant de l'introduction dans le gène codant pour la sous-unité d'un pilus, d'au moins un fragment d'ADN codant pour un piptide ou une protéine hétérologue tel qu'un fragment d'ADN synthétique, un fragment d'ADN naturel ou un fragment obtenu à partir de la transcription réverse de mRNA. Par ADN hétérologue, on entend tout matériel génétique issu d'une autre source que la souche hôte dans laquelle on réalise le procédé de l'invention. Par ADN synthétique, on entend aussi bien un ADN de composition ou structure naturelle obtenu par synthèse chimique qu'un ADN dont la structure ou la composition n'existe pas tel quel dans la nature.

De manière préférée ces changements résultent de l'introduction dans le gène codant pour la sous-unité d'un pilus, d'au moins deux fragments d'ADn codant pour un peptide ou une protéine hétérologue, ceux-ci pouvant être identiques ou différents.

Un des procédés est la modification d'au moins une région de la protéine de la sous-unité formant le pilus exposée au milieu environnant, par insertion d'ADN codant pour un peptide ou une protéine hétérologue, dans au moins une zone du gène codant pour la sous-unité d'un pilus correspondant à la zone exposée au milieu environnant de la séquence protéinique des pilines concernées.

Les régions exposées comprennent notamment des régions antigéniques des pili, c'est-à-dire composées d'épitopes inducteurs de la production d'anticorps ou reconnus par des anticorps.

L'invention concerne également les utilisations des pili et pilines porteurs de matériel hétérologue. Ainsi

4

l'invention concerne notamment les vaccins obtenus avec les pili et pilines porteurs de matériel hétérologue, combinés ou non avec divers additifs tels que des adjuvants et des immunostimulants.

L'invention concerne également l'utilisation des pili, des pilines et des microorganismes ayant une membrane externe qui porte des pili, dont la composition est modifiée par au moins un changement, ou par au moins deux changements, de la séquence protéique des pilines; en particulier l'invention concerne les utilisations de ceux-ci pour l'obtention d'un produit pharmaceutique destiné au traitement médical et/ou prophylactique en santé humaine ou animale.

L'invention concerne également les tests immunologiques obtenus à partir des pili et pilines porteurs de matériel hétérologue.

L'invention concerne notamment l'utilisation des pili, des pilines et des microorganismes ayant une membrane externe qui porte des pili, dont la composition est modifiée par au moins un changement, ou par au moins deux changements, de la séquence protéique des pilines, pour la fabrication de moyen destiné à la préparation de tests immunologiques. Ceci inclut notamment les composants de tests diagnostiques, les tests d'analyse, les galeries d'identification, les biopsies, les divers examens biologiques, les kits d'analyse et de dosage, les bandelettes pour le diagnostic, les réactifs diagnostiques.

L'invention comprend également des utilisations diverses dans lesquelles les pili porteurs de protéines ou de peptides sont utilisés en tant qu'agents ayant des propriétés immunostimulantes (permettant notamment la stimulation de la réponse immunitaire à partir de vaccins déjà connus).

Un procédé ayant donné de bons résultats fait appel aux techniques du génie génétique. Il comprend les étapes suivantes :

(1) clonage du gène ou de l'opéron du pilus choisi comme protéine porteuse et l'expression de ce(s) gène(s) dans un organisme hôte,

(2) détermination de la séquence du gène de la piline

(3) alternative de la complémentation intergénique,

(4) identification des sites antigéniques de pilus,

(5) identification des régions variables telle que notamment par comparaison des séquences de différents sérotypes,

(6) isolement (ou synthèse) d'un fragment d'ADN codant pour un peptide ou un épitope intéressant, caractéristique de l'organisme contre lequel on veut dresser des anticorps,

(7) clonage de l'ADN obtenu en (6) en phase dans le gène de la sous-unité du pilus dans des sites préférentiels identifiés en (4) et (5),

(8) introduction du gène recombinant dans un organisme et son expression en tant que pilus porteur de peptide ou de protéine hétérologues.

(9) purification des pili porteurs de peptide ou de protéine hétérologues.

Ces étapes sont explicitées ci-après :

## 1. Clonage

Le clonage de l'opéron ou du gène du pilus choisi comme molécule porteuse est réalisé par les techniques maintenant classiques du génie génétique.

Le clonage in vitro implique la préparation de l'ADN de l'organisme qui produit le pilus, et le clonage de fragments de restriction (banque génomique) dans des plasmides, des phages ou des cosmides.

L'emploi de vecteurs dits d'expression, contenant des signaux de transcription forts, peut pallier l'absence de promoteur sur le fragment de DNA cloné.

Le clonage in vivo peut se faire chez certaines bactéries par l'intermédiaire d'un phage transducteur ou de plasmides mobilisables ou transférables associés à des transposons.

Les vecteurs de clonage sont introduits dans un organisme hôte par transformation (plasmides) ou transfection (cosmides et phages) ou conjugaison (plasmides conjugatifs).

L'expression des pili est recherchée dans un organisme hôte ne produisant pas ce type de pili : soit un mutant de l'organisme, muté dans cette fonction ou, mieux, dans un organisme de laboratoire, tel E. coli K12 pour les procaryotes. Le criblage des recombinants se fera sur base des propriétés propres aux pili (telles que l'hemagglutination, l'immunoprécipitation, ou la reconnaissance immunologique sur colonie). La présence des pili est confirmée de visu par microscopie électronique, ou par la purification des pili d'une culture du recombinant et leur analyse sur gel d'électrophorèse par exemple.

## 2. Séquençage du gène

Une carte de restriction du fragment cloné est établie pour quelques enzymes courants. On utilise pour cela les techniques de restriction partielle et de restrictions multiples.

Le séquençage de l'ADN cloné est réalisé soit par la technique dite du didéoxyribose développée par Sanger, soit la technique de modification des bases mise au point par Maxam et Gilbert.

On positionne le gène de la sous-unité sur la carte de restriction par des techniques de mutagenèse. Un exemple d'exécution particulière de cette technique fait appel aux minicellules. Ces minicellules permettent de mettre en évidence les protéines codées par le fragment cloné sur un plasmide. Il est possible d'induire des mutations dans ce fragment, par exemple via l'intégration d'un transposon, ou in vitro par restriction, suivie de digestion par une exonucléase. Les mutations qui entraînent l'absence de synthèse de pili sont analysées à leur tour dans les minicellules : on associe ainsi l'absence d'une protéine produite avec la localisation de la mutation. L'absence de sous-unités positionne, sans équivoque, le gène de la sous-unité sur le fragment. Cette portion du fragment peut être seule séquencée.

La séquence est analysée par ordinateur, via des programmes d'analyse qui fournissent les données essentielles de :
- début et fin du (des) gène(s)
- traduction du gène en protéine
- position des sites de restriction.

### 3. Complémentation intergénique

La plupart des pili dont la génétique a été décrite à ce jour sont structurés en opéron, constitué de plusieurs gènes en plus du gène de la sous-unité. Ces gènes codent par exemple pour des protéines jouant un rôle dans l'ancrage, l'assemblage ou la protection.

On peut fractionner l'opéron en clonant le seul gène de la sous-unité sur un plasmide et le reste sur un autre plasmide. Les deux plasmides vecteurs doivent être compatibles et porter des marqueurs de sélection différents. Les pili seront synthétisés seulement lorsque les deux plasmides seront présents dans l'organisme hôte, par complémentation intergénique.

Le fragment portant l'opéron délété du gène de la sous-unité peut également être introduit dans le chromosome de l'organisme hôte.

La complémentation intergénique a plusieurs avantages techniques fort intéressants.

Le plasmide contenant le gène de la sous-unité
- est seul à devoir être manipulé,
- est d'une taille réduite par rapport à celle du plasmide porteur de l'opéron complet,
- contient un nombre plus limité de sites de restriction,
- a été complètement séquencé, ce qui n'est pas toujours le cas pour l'entièreté de l'opéron.

### 4. Sites antigéniques

En disposant d'anticorps polyclonaux ou d'une batterie d'anticorps monoclonaux spécifiques des pili, on recherche la position des antigènes par différentes techniques : par exemple

a - à partir de peptides synthétiques du pilus, qui sont reconnus par des anticorps antipili, ou qui induisent, après injection à un animal, la synthèse d'anticorps antipili.

b - par fragmentation de la protéine par des composés comme le bromure de cyanogène ou des enzymes (trypsine, ...) qui clivent les sites spécifiques de la protéine. On cherche ensuite la présence d'antigènes sur les différents fragments.

c - par clonage de fragments du gène pilus en phase dans le gène de la bêta-galactosidase. Si le fragment cloné détermine un peptide antigénique, il sera reconnu sur la protéine hybride par les anticorps antipili.

Ces techniques identifient des épitopes continus de la protéine.

Des données prédictives sont souvent utilisées pour localiser des épitopes :

a - La structure tridimensionnelle de la protéine, si elle est connue, identifie les régions exposées.

b - Des algorithmes peuvent être utilisés pour :
- rechercher les régions hydrophiles et les régions hydrophobes d'une protéine : algorithmes de Hoop & Wood, de Doolittle. Les régions hydrophiles, exposées au solvant, sont autant de lieux pour des épitopes potentiels.
- prédire la structure secondaire (hélice, feuillet et tours) d'une protéine à partir de sa seule séquence primaire : algorithmes de Chou & Fasman, de Garnier. Les épitopes sont souvent associés à des tours dans la protéine.

D'autres indices peuvent être relevés, comme la présence de résidus proline, un acide aminé qui provoque des "coudes", et donc des régions protubérantes dans la protéine.

## 5. Identification des régions variables

Les régions dites "variables" de la piline sont, d'une manière générale, les régions, dont la séquence en acides aminés peut être modifiée, sans compromettre la fabrication des pili.

Une manière expérimentale de localiser les régions variables consiste à cloner une courte séquence d'ADN synthétique au hasard dans le gène de la piline, puis de sélectionner les bactéries qui restent piliées. La position de l'insertion est déterminée par le séquençage du gène. Il positionne des régions variables de la piline.

Une manière prédictive consiste à comparer les séquences en acides aminés des pilines dans les différents sérotypes. En effet, certains pili présentent des variations antigéniques naturelles plus ou moins importantes, permettant sans doute à l'organisme qui les porte d'échapper partiellement du moins, à l'immunité acquise par l'hôte lors d'une infection antérieure.

En comparant les séquences en nucléotides et en acides aminés des différents variants, on peut reconnaître :

a) des régions invariables, communes à tous les sérotypes, qui seront considérées comme essentielles à la structure ou la polymérisation de la piline. La présence de mutations conservatrices est un indice supplémentaire qui accentue l'importance de ces régio la structure pili;

b) des régions variables, en composition en acides aminés et/ou en longueur. Ces régions seront considérées comme n'intervenant pas - ou peu - dans l'essemblage et la structure du pilus.

Cette première analyse peut être plus fouillée, tenant compte notamment des propriétés chimiques des résidus variables, de leur indice d'hydrophilicité ou encore de la taille du radical. L'importance des variations entre les pili peut ainsi être pondérée.

Les épitopes positionnés comme décrit au point ci-avant en 4) peuvent être caractérisés grâce à la comparaison de sérotypes, comme communs ou variables. Cette donnée n'est pas indispensable pour la bonne réalisation de notre invention, mais est cependant fort intéressante. En effet, elle définit sur la protéine des régions à la fois antigéniques et susceptibles de modifications, qui seront autant de régions cibles pour l'introduction de peptides étrangers.

## 6. Epitope

Il faut disposer d'un fragment d'ADN qui détermine la synthèse d'un épitope particulier de l'organisme contre lequel on désire développer une réponse immunologique. Ce fragment est soit un fragment de restriction, soit un ADN synthétique. Cette seconde solution offre évidemment beaucoup plus de souplesse pour le clonage (cf. point 7).

## 7. Clonage d'ADN

L'ADN codant pour l'épitope est cloné, en phase, dans le gène de la sous-unité, dans une des régions antigéniques et variables établies en (4) et (5) ci-avant. Cet ADN est soit ajouté au gène, soit cloné en lieu et place d'un fragment existant. Dans ce dernier cas, la taille totale de la sous-unité pourra être maintenue.

La stratégie de clonage dépend de la disponibilité de sites de restriction dans la région choisie. En cas d'absence d'une telle séquence, un site est créé par mutagenèse in vitro, ou éventuellement l'ADN est cloné par des techniques de mutagenèse in vitro. L'introduction d'un site de restriction unique présente le grand avantage de créer un vecteur universel. En effet, n'importe quel ADN synthétique possédant des extrémités cohésives avec la séquence restreinte pourra être dorénavant cloné dans ce site.

## 8. Expression du gène recombinant

Le vecteur portant le gène recombinant est introduit dans l'organisme hôte capable de produire le pilus modifié, notamment l'organisme porteur du système de complémentation décrit en 3. La présence de pili est mise en évidence comme lors du criblage décrit en (1), alors que des anticorps spécifiques de l'épitope cloné peuvent en prouver la présence.

## 9. Purification

Les pili recombinants sont extraits des cultures du microorganisme hôte, puis purifiés.

Deux techniques d'extraction ayant donné de bons résultats sont l'extraction mécanique et l'extraction thermique.

Les pili sont ensuite purifiés par toute méthode connue, telles que notamment par ultrafiltration, précipitation et chromatographie.

Exemples de réalisation

Des exemples de réalisation particulière de l'invention sont décrits ci-après, ils illustrent l'invention sans en limiter la portée. Ces exemples sont réalisés avec les pili K88 d'Escherichia coli.

Les pili K88 sont des pili somatiques produits par des Escherichia coli entérotoxigéniques. Ils leur permettent d'adhérer aux cellules épithéliales de l'intestin et de coloniser ce milieu. E. coli K88 existe sous trois variations sérotypiques : chaque variant possède un type antigénique commun, noté a, et un type variable, noté respectivement b, c et d.

Exemple 1

Le procédé suivi dans cet exemple peut être décrit de la façon générale suivante :

a - La cartographie des épitopes du pilus d'une part et la comparaison des séquences des trois sérotypes ab, ac et ad d'autre part permettent de localiser des régions antigéniques et var iables du pili.

L'analyse des séquences de l'ADN fait apparaître des sites de restriction utilisables pour les clonages.

b - le fragment d'ADN déterminant un peptide antigénique reconnu par un anticorps donné est introduit par manipulation génétique dans le gène de la sous-unité du pilus K88. Le site d'insertion est choisi de façon à ce que le peptide hétérologue conserve les propriétés d'excrétion et d'assemblage du pilus.

c - Le gène recombinant est introduit dans une bactérie qui l'exprime et assemble les pilines en pili. Le peptide antigénique qui est fusionné à la sous-unité est donc multiplié sur le pilus.

d - Les pili sont ensuite purifiés. Ils sont décrochés des membranes par l'agitation d'une suspension de bactéries piliées dans un mixer. Pili et bactéries sont séparés par centrifugation.

Résultats expérimentaux

A - Matériels et méthodes

1 - Souches bactériennes et plasmides

Les souches bactériennes utilisées sont :

EC294 : End I⁻, hsd (r̄$_k$, m̄$_k$), sup E

JM103 : (Lac, Pro), Thi, strA, endA, sbcB15, hsdR⁻, supE, recA/F′, traD36, proAB, lac I $^q$, LacZΔM15.

K88abNL - souche E. coli K88$^+$, sérotype ab, reçue du Dr. Van Zijderveld (CDI, Lelystad, NL)-

K88abUS - idem, reçue de Salsbury Laboratories, USA -

1476 pK88ac souche E. coli K88$^+$, sérotype ac, reçue de Salsbury Lab.

K88adNL - sérotype ad, reçue du Dr. Van Zijderveld.

K88adUS - idem, reçue de Salsbury Lab.

Les souches d'Escherichia coli portant la référence 1476(pK88ac) et K88adUS ont été déposées à la N.C.I.B. (National Collections of Industrial & Marine Bacteria Ltd - Aberdeen - Ecosse). Ces dépôts ont été réalisés conformément au Traité de Budapest. La souche d'Escherichia coli 1476 (pK88ac) a reçu le numéro NCIB 12346. La souche d'Escherichia coli K88adUS a reçu le numéro NCIB 12348.

Les plasmides utilisés et construits durant ce travail sont décrits dans le tableau I, par référence leur nom débute par la lettre p.

Les cultures sont réalisées en milieu Luria broth (LB), additionné des antibiotiques appropriés aux concentrations de 100 micro g/ml pour l'ampicilline, 25 micro g/ml pour la tétracycline et le chloramphenicol. Le caractère F$^+$ de JM103 est testé par sa sensibilité au phage M13 comme décrit par Miller (Experiments in Molecular Genetics, Cold Spring Harbor Laboratory, 1972). Les auxotrophies sont testées par la croissance sur milieu minimal, additionné des acides aminés (aa) adéquats.

La production de beta-galactosidase, phénotype Lac, est mise en évidence par la coloration bleue des colonies sur un milieu LB contenant de l'isopropyl-beta D(-)thiogalactoside (IPTG) (425 micro g/ml final) et du 5-bromo-4-chloro-3 indolyl beta D galactopyranoside (X gal) (40 micro g/ml final).

## 2. Enzymes

Les enzymes de restriction, phosphatase (CIP), T4-polymerase et le fragment Klenow sont utilisés selon les recommandations des manufacturiers. La T4-ligase est employée à $10^{-2}$ u/micro g d'ADN pour la ligation des bouts cohésifs et à 1 u/micro g d'ADN pour la ligation d'extrémités aveugles.

Bal 31 à la concentration de $5.10^{-2}$ u/ micro g d'ADN enlève 600 bp par min.

La RNase est à 20 micro g/ml finale. Les conditions de digestion par la DNase sont de 10 micro g DNase/micro g DNA linéaire pendant 15 min.

## 3. Préparation d'ADN

Les plasmides sont purifiés par la méthode du lysat alcalin sur 5 ml de culture (mini préparation). La technique du lysat clarifié suivie d'un gradient de CsCl est utilisée pour la purification de plasmides à partir d'un litre de culture (cf. Maniatis et al, 1982, Molecular cloning, a laboratory manual, Cold Spring Harbor Laboratory).

Hormis le plasmide pACYC184 et ses dérivés, les plasmides sont amplifiés au chloramphenicol (75 micro g/ml fin qui est ajouté lorsque la culture atteint la densité optique de 0,7.

## 4. Transformation

La technique au CaCl$_2$ est utilisée pour la transformation d' E. Coli (cf. Maniatis et al, 1982).

## 5. Electrophorèses

Pour l'analyse de l'ADN, les électrophorèses d'agarose sont réalisées sur des appareils horizontaux, tampon tris-acétate (Tris 40 mM, acétate 20 mM, EDTA 1 mM pH 8.0), ou tris-borate (Tris 90 mM, acide borique 90 mM, EDTA 2 mM, pH 8.0) en présence de bromure d'éthidium. Le standard de taille est l'ADN du phage lambda restreint par HindIII.

Les gels d'acrylamide-bisacrylamide (38:2) sont réalisés dans du tampon tris-borate.

Pour l'analyse des protéines, les gels SDS-PAGE sont à la concentration de 15 % en polyacrylamide. Les électrophorèses sont réalisées selon la technique classique décrite par Laemmli (1970 -Nature, 227, 680-685).

## 6. Séquençage

Les fragments d'ADN marqués en 5′ à une extrémité sont séparés sur gel d'agarose ou sur gel d'acrylamide et les fragments nécessaires au séquençage sont élués. La procédure de séquençage décrite par Maxam et Gilbert (1980, Methods in Enzymology, 65, 499-560) leur est appliquée.

## 7. Techniques immunologiques

La présence de pili K88 sur des bactéries adsorbées sur un filtre de nitrocellulose est révélée par une méthode immunologique en utilisant un antisérum polyclonal anti-K88 et des immunoglobulines IgG anti-lapins couplés à la peroxydase. Le substrat est le 4-chloronaphtol.

La détection de peptides antigéniques de K88, fusionnés à la bêta-galactosidase suit la méthode immunologique sur colonie décrite par Stahl et al (1984, Proc. Nath. Acad. Sci., 81, 2456-2460).

Les protéines séparées sur gel SDS-PAGE sont transférées sur une membrane de nitrocellulose (appareillage Transblot de BioRad); qui est ensuite soumise à un test immunologique avec divers anticorps spécifiques (technique dite du "Western Blot").

L'ELISA sandwich est réalisé selon la technique décrite par Engvall (1980, Methods in Enzymology, vol. 70, 419-439), en utilisant des anticorps anti-K88 mono- ou polyclonaux.

## 8. Constructions génétiques

Les techniques utilisées pour les diverses constructions génétiques sont décrites par Maniatis et al, 1982.

## 9. Préparation des pili

Les pili sont extraits selon deux techniques l'une préparative qui emploie l'omnimixer, l'autre analytique qui fait intervenir un traitement à la chaleur.

a. Méthode préparative

Un litre de culture liquide de bactéries piliées est centrifugée 10 min à 7000 g ($62.10^3$ radian.$s^{-1}$) et le culot est repris dans 1/40 du volume initial de solution PBS urée (25 mM $NaH_2PO_4$, 5 mM $Na_2HPO_4$, 25 nM NaCl, 2M urea, pH 7.4).

L'extraction est effectuée par une agitation 30 min dans un omnimixer Sorvall, en position 5. Le produit d'extraction est centrifugé pendant 10 min à 7000 g ($62.10^3$ radian.$s^{-1}$) (rotor JA20 - Beckman). Le surnageant constitue le rasat brut.

Les pili peuvent être purifiés par une précipitation acide (acide acétique 1M, pH4.5, 1 h à température ambiante) suivie d'une chromatographie sur gel de perméation (gel de séphacryl, Pharmacia S200). Cette solution constitue le rasat purifié, il peut être concentré et purifié par ultrafiltration.

b. Méthode analytique

100 ml de culture sont centrifugés et le culot est remis doucement en suspension dans 10 ml de PBS (Phosphate buffered saline) préchauffé à 60°C. Les bactéries sont maintenues dans un bain à 60°C, sans agitation, pendant 20 min. puis centrifugées pendant 10 min à 7000 g ($62.10^3$ radian.$s^{-1}$).

Le surnageant constitue le rasat brut.

Cette méthode d'e xtraction sera employée pour la première analyse et la quantification des pili que l'on peut extraire des cultures des différents recombinants.

B. RESULTATS

Les pili excrétés par les souches E. coli K88 sauvages, appartenant aux trois sérotypes ab, ac et ad, et provenant soit de Hollande (notation NL) ou des Etats-Unis (notation US) ont été extraits et séparés sur gel SDS-PAGE. La figure la montre un tel gel coloré au bleu de Coomassie. Les sous-unités pilines forment une bande électrophorétique dont la migration est celle d'une protéine de 26 kD.

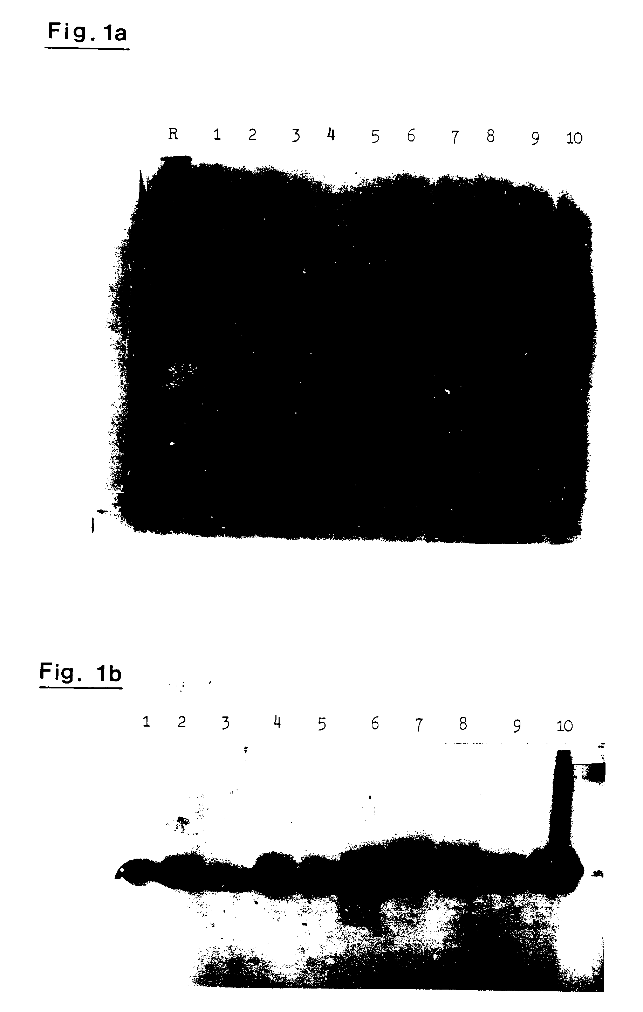

La figure 1b présente un western blot du gel de la figure 1a. Les anticorps polyclonaux dressés contre les pili du sérotype ac reconnaissent sans équivoque les pilines des trois sérotypes.

Les protéines appliquées dans les autres puits seront décrites par la suite.

1. Clonage de K88

a. Sérotype K88ac

L'opéron K88 est localisé sur un plasmide conjugatif, appelé pK88ac.

Le plasmide pK88ac de 80 kb, transféré dans la souche d'E. coli 1476 $Nal^R$, a été purifié, puis restreint par l'endonuclease HindIII. Les fragments HindIII sont clonés dans le vecteur pBR322, restreint par HindIII et traités à la phosphatase, de manière à limiter sa recircularisation.

L'insertion d'un fragment dans le site HindIII inactive la résistance à la tétracycline. Trois cents recombinants résistants à l'ampicilline et sensibles à la tétracycline ($Ap^R Tc^S$) ont été testés pour la présence de pili K88 par agglutination sur une plaque de verre provoquée par un antisérum K88. Neuf donnent une réaction positive, dont cinq présentent une agglutination rapide, comparable à celle provoquée par la souche sauvage, et quatre présentent une agglutination lente.

Les neuf clones possèdent un plasmide contenant un fragment de restriction d'une taille de 12 kb. L'orientation de l'insert est antihorlogique pour les 5 clones nettement positifs et horlogique pour les 4 autres. La dépendance de l'expression de K88ac vis-à-vis de l'orientation peut s'expliquer par la présence du promoteur cryptique P1, situé en aval du site HindIII, connu pour déterminer une transcription dans le sens antihorlogique (Stuber et Bujard, 1981, Proc. Natl, Acad. Sci, 78, 167-171). La forte expression de l'opéron K88 dépend donc d'un promoteur externe au fragment HindIII cloné.

L'un des plasmides des 5 clones nettement positifs a été choisi, noté pIX102, et manipulé plus avant.

La taille de l'insert a été réduite à 7kb par restriction partielle EcoR1 (plasmide pIX105). Le site EcoR1 a été remplacé par un site HindIII dans le plasmide pIX115, de telle sorte que l'opéron K88 soit sur un fragment HindIII.

Les figures 1a et 1b, piste 5, montre les protéines extraites d'une culture d'E. coli porteuse du pIX115.

La sous-unité a la même migration que celle produite par la souche ac sauvage, et est clairement reconnue par les anticorps anti-K88ac.

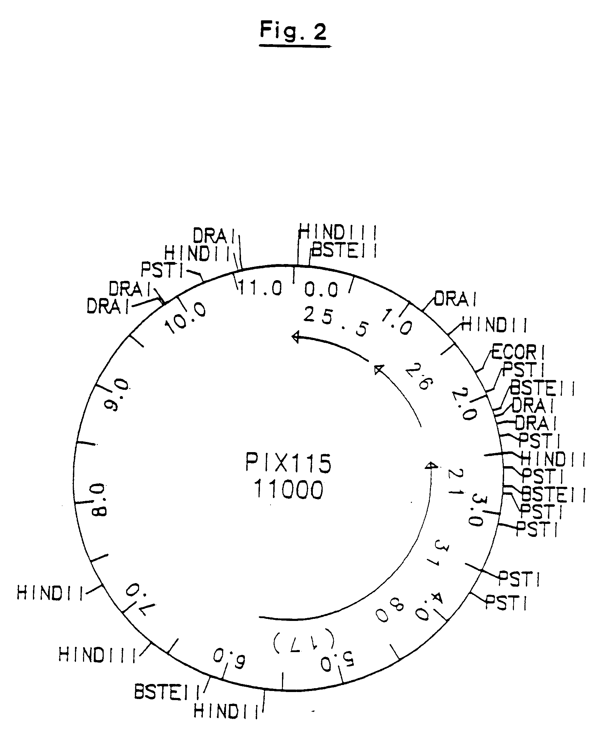

Une carte de restriction sommaire du pIX 115 est présentée à la figure 2, ainsi que la carte génétique de l'opéron K88ac. La position relative des différents gènes est déduite essentiellement des données de la littérature (Dougan et al, 1983, J. Bacteriol., 153, 364-370) pour le sérotype K88ac et, par analogie avec K88ac pour le sérotype K88ab (Mooi et al, 1983, J. Bacteriol., 154, 41-49). Le poids moléculaire de différentes protéines produites par l'opéron K88 est de 80, 31, 21 kD, et 26 kD pour la sous-unité. Une protéine de 25 kD pourrait être codée par le dernier gène de l'opéron.

La production d'une protéine de 17,6 kD n'a pas été prouvée par des expériences in vivo, mais déduite de la séquence de l'ADN.

Le gène de la sous-unité K88ac est sur un fragment DraI, de 1 kb environ. Le fragment a été cloné dans le site HindIII du pBR322, dans les deux orientations. Le plasmide poss édant le gène sous le contrôle de P1, soit l'orientation antihorlogique, est noté pIX120.

D'autre part, le gène de résistance à la tétracycline a été délété après restriction par BamHI et AvaI, suivie d'un traitement à la DNA polymérase en présence des quatres dNTP et ligation. Cette délétion maintient un site AvaI dans le plasmide pIX120.

### b. K88ad

L'ensemble des plasmides de la souche K88ad US ont été purifiés et restreints par HindIII. Les fragments ont été séparés sur gel d'agarose 0.7 % et les fragments de 10 à 15 kb ont été électroélués et clonés dans le vecteur pBR322 restreint par HindIII et déphosphorylé à la phosphatase CIP.

La production de K88ad par des clones E. coli transformés est recherchée par un test immunologique sur colonie (cf. Méthode). Les clones K88[+] possèdent un insert de 12 kb dans l'orientation antihorlogique et le plasmide est noté pAD102.

La production de pili K88ad par la souche E. coli (pAD102) est confirmée par un gel d'électrophorèse et Western blot d'un rasat brut d'une culture liquide (cf. figure 1, piste 9).

Le fragment de 1 kb BstEII-DraI du pAD102 a été cloné dans les sites BstEII-EcoRI Klenow du pIX120, générant ainsi le plasmide pAD120. De la même manière que pIX120, le pAD120 contient le gène de structure de la sous-unité piline du sérotype K88ad. L'insertion antihorlogique de l'insert dans le site HindIII place le gène sous le contrôle du promoteur fort P1.

### 2. Séquençage

Les gènes des sous-unités K88ac (pIX120) et K88ad (pAD120) ont été séquencés sur les deux brins, par la technique de Maxam et Gilbert. Les sites de restriction marqués par la Klenow ont été les sites HinfI, DdeI et EcoR1 notamment.

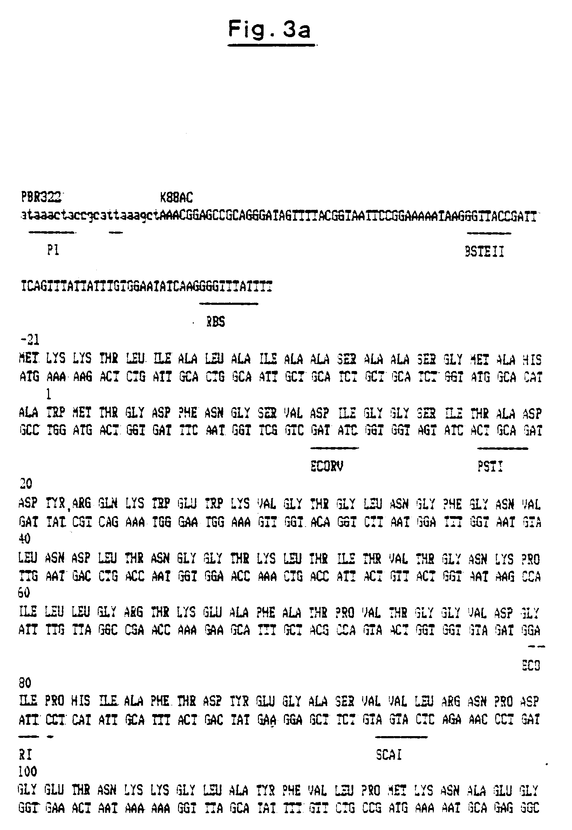

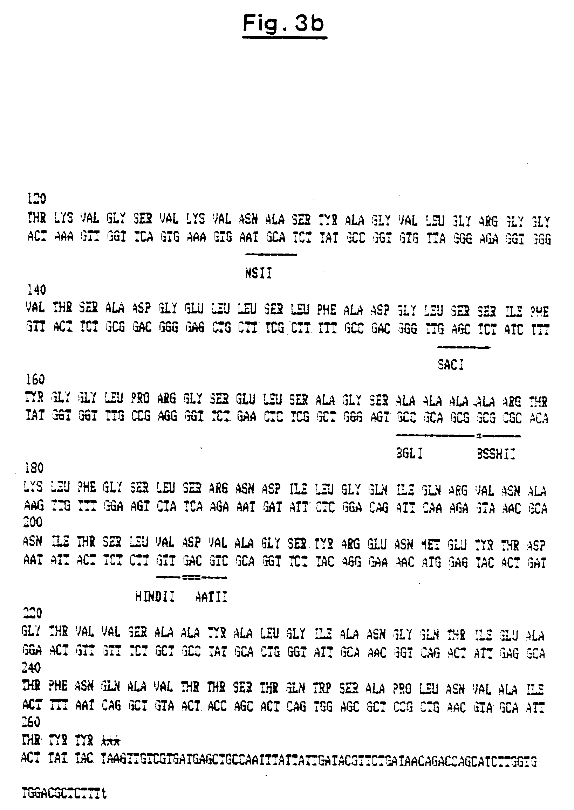

La séquence complète de l'insert du pIX120, tel qu'il est cloné dans le site HindIII du pBR322 est donnée à la figure 3.

Cette figure montre qu'il existe une phase de lecture ouverte en -63 codant pour un ATG initiateur, terminée en 710 par un codon stop TAA. Cette phase de lecture code pour la sous-unité piline. Celle-ci est synthétisée sous la forme d'un précurseur portant une séquence signal de 21 acides aminés à l'extrémité amine de la protéine. Cette séquence permet au précurseur de traverser la membrane interne de la bactérie, et serait ensuite clivée.

La sous-unité est composée de 262 acides aminés formant une protéine d'un poids moléculaire (PM) déduit de 27.337, proche des 26 kD déterminés expérimentalement. Les séquences N et C terminales déduites de la séquence du gène sont identiques aux séquences de la protéine du sérotype K88ab.

Une séquence complémentaire au RNA 16S est présente 14 paires de bases en amont de l'ATG et devrait constituer une séquence efficace d'attachement au ribosome (RBS).

Le promoteur P1 du pBR322 est souligné sur la figure 3. Aucune autre séquence qui puisse correspondre à un promoteur n'est observée en amont du RBS du fragment K88.

La position de quelques sites de restriction est également donnée. Epinglons des sites de restriction uniques tels EcoRV, Pst1, EcoR1, ScaI, Nsi1, Sac1, Bgl1, BsHII, HindII et Aat2.

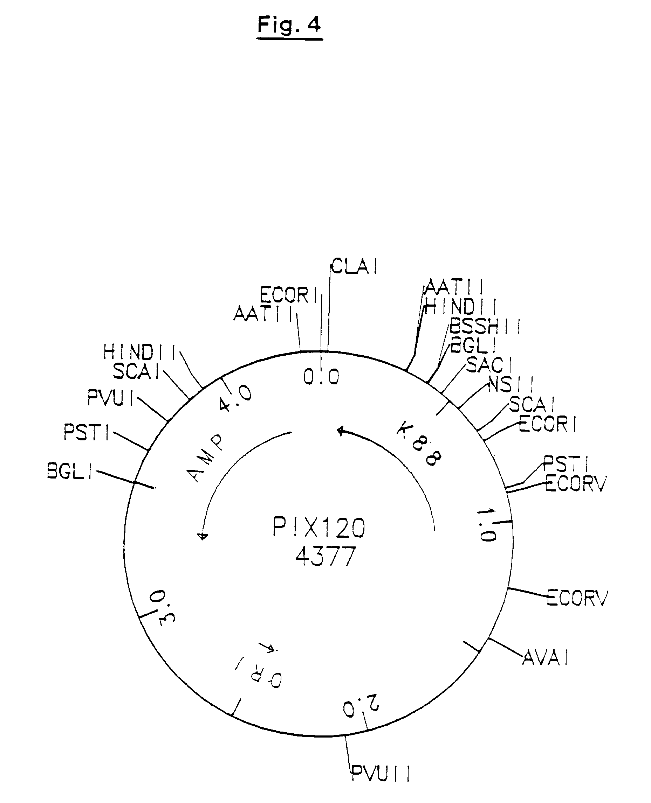

La figure 4 montre la carte de restriction du pIX120.

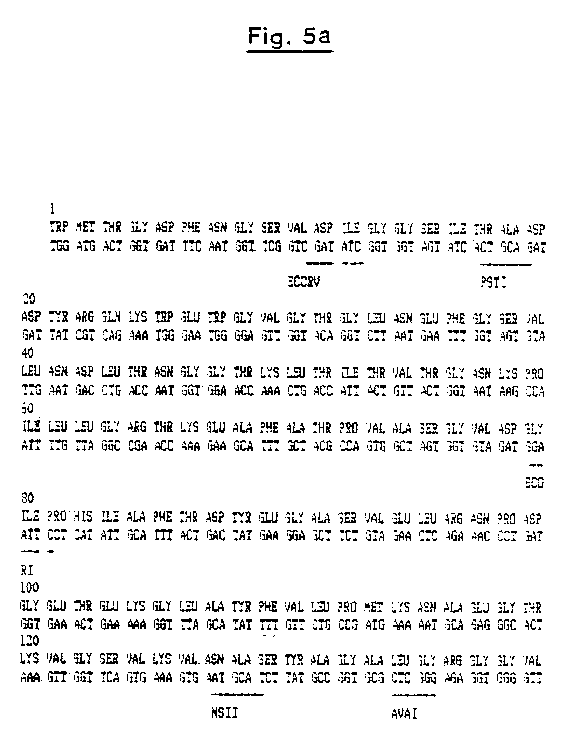

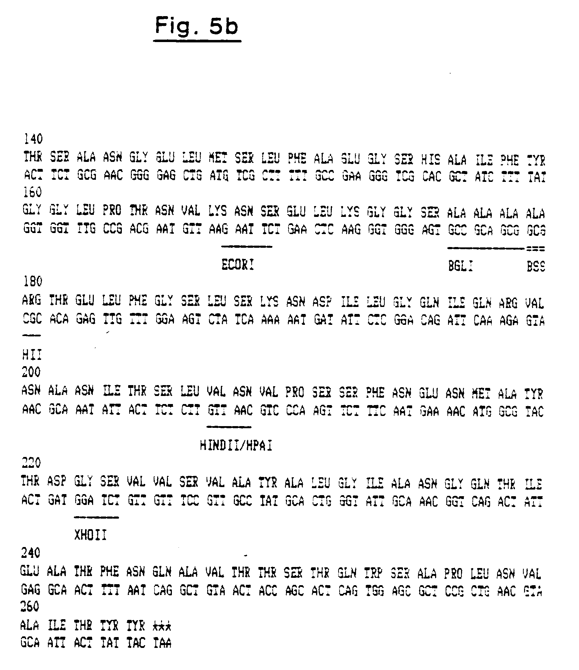

La séquence complète du gène de la sous-unité K88ad est donnée à la figure 5. Cette sous-unité est composée de 264 résidus, pour un poids moléculaire déduit de 27.535.

Le gène de la sous-unité K88ad US contient des sites de restriction différents de ceux observés dans le gène ac. La présence des sites AvaI, HpaI, et XhoII, l'absence des sites ScaI et SacI et l'existence de deux sites EcoR1 ont été retenues.

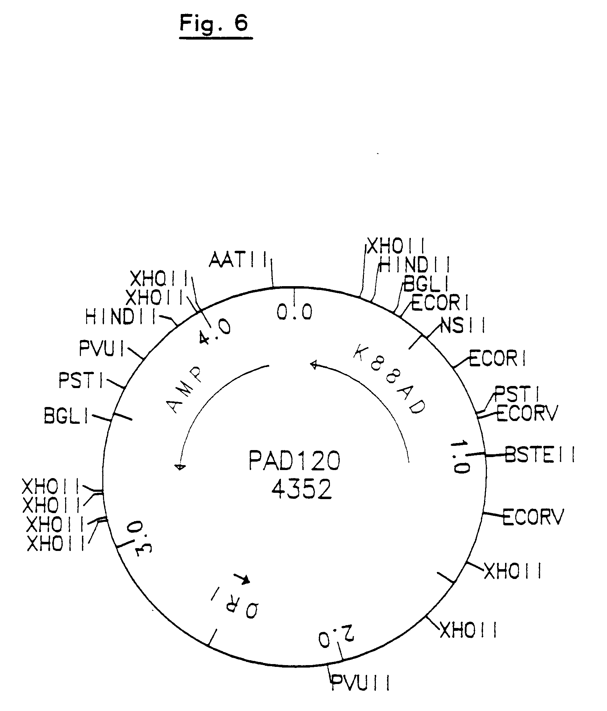

La carte de restriction du pAD120 est donnée à la figure 6.

3. Complémentation intergénique

a. Pour K88ac

L'opéron K88ac est composé de 5 gènes, dont le gène de la sous-unité et quatre gènes dont les produits s ervent à l'ancrage du pilus et à sa polymérisation (cf. figure 2). Ces 5 gènes ont été répartis sur les vecteurs de clonage pACYC184 et pBR322. Ces deux vecteurs sont compatibles, portent des marqueurs de résistance différents (au chloramphenicol pour pACYC184 et à l'ampicilline pour pBR322), et sont tous deux à grand nombre de copies par cellule. De plus, ils ont le même gène de résistance à la tétracycline, donc le même promoteur P1 localisé en amont du site HindIII.

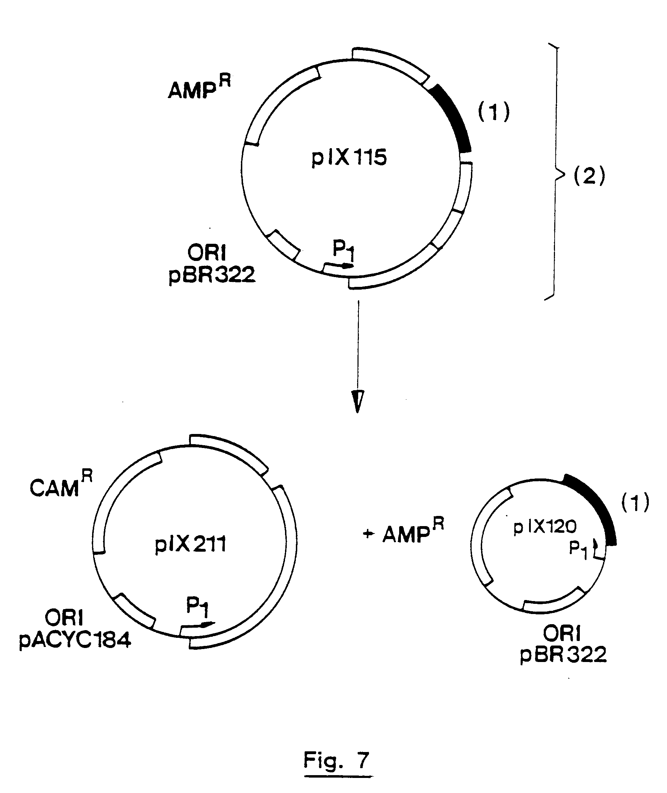

La complémentation de K88ac est schématisée à la figure 7. Elle fait intervenir les 2 plasmides pIX120 et pIX211 construits de la manière suivante :

(1). pIX120

Le pIX120 contient le seul gène de la sous-unité K88ac sous le contrôle de P1 dans le pBR322. Le gène $Tc^R$ a été délété pour éviter la recombinaison des gènes homologues $Tc^R$ entre les vecteurs pBR322 et pACYC 184 dans un environnement $Rec^+$.

(2). pIX211

Le pIX211 est un plasmide pACYC184 contenant l'opéron K88ac délété du gène de la sous-unité, et cloné dans le site HindIII, donc sous le contrôle du promoteur P1. Ce plasmide a été construit en plusieurs étapes :
- isolement du pBR322E, qui est un pBR322 dépourvu de site EcoR1. Il a été obtenu par restriction EcoR1 du pBR322, et traitement à la Klenow.
- clonage de l'operon K88 dans le site HindIII du pBR322E. Ce plasmide est l'équivalent du pIX115, portant un seul site EcoR1, localisé au premier tiers du gène de la sous-unité (cf. figure 2).
- linéarisation du plasmide par EcoR1, suivie d'une digestion de 600 à 1000 paires de bases par Bal 31. Les plasmides sont ligaturés après avoir été rendus "blunt ended" par la T4 DNA polymérase. L'importance de la délétion des recombinants est estimée par restriction BstEII, PstI et DraI (Cf. position de ces sites sur la figure 2.)
- Quelques recombinants ont servi à transformer une souche E. coli contenant le seul gène de la sous-unité cloné dans le site HindIII du vecteur pACYC184. La fabrication de pili par les clones $Cm^R$, $Ap^R$ est testée sur colonie par méthode immunologique.
- Le plasmide qui a subi la plus grande délétion, tout en permettant la complémentation a été finalement sélectionné. Cette délétion est d'environ 1000 paires de bases; elle couvre les sites BstEII, localisé 50 bp avant le codon initiateur de la sous-unité, et le site HindII, situé 110 bp avant le codon stop. La séquence RBS et le gène complet de la sous-unité sont donc délétés, sauf peut-être les derniers codons du côté 3′ du gène.
- La dernière étape est le clonage du fragment HindIII de ce plasmide dans le site HindIII du pACYC184. Ce plasmide est le pIX211.

Les bactéries qui contiennent à la fois le plasmide pIX120 et le plasmide pIX211 sont $K88^+$.

Un extrait d'un rasat brut d'une culture de EC294 (pIX211) (pIX120) est présenté à la figure 1, piste 6.

b. Pour K88 ad

Les souches qui contiennent le pAD120 - porteur du gène de la sous-unité K88ad - et le pIX211 - porteur du système d'assemblage de K88ac-, fabriquent du pili K88ad, comme le montre la présence de piline ad dans un rasat brut d'une culture de cette souche (figure 1, piste 10). Le système de complémentation mis au point pour K88ac peut donc être utilisé pour le sérotype ad.

4. Recherche des épitopes de K88

a. Données expérimentales

12

Le principe de la recherche d'épitopes est le clonage de fragments du gène de la sous-unité dans le gène de la beta-galactosidase. La présence d'un peptide K88 antigénique fusionné à la beta-galactosidase est recherchée par un test immunologique sur colonie, avec l'antisérum spécifique du pilus.

Cette technique a été appliquée au sérotype K88ac de la manière suivante :

Le segment BstEII - Dra1 du pIX115, contenant le gène de la sous-unité (cf. figure 2), est hydrolysé par la DNase, de manière à isoler des fragments de 50 à 150 paires de bases. Les fragments sont remplis à la T4-polymérase en présence des 4dNTP et clonés dans le site HindII (extrémités aveugles) du pUR222. Le site HindII est unique et fait partie d'un polylinker, situé dans le fragment Lac alpha de la beta-galactosidase. Les plasmides sont introduits dans la souche JM103, qui possède le plasmide F' porteur de l'opéron lactose complet, mais muté dans la région lac alpha. Le phénotype lac[+] apparaît par complémentation entre les gènes portés par les deux plasmides, et est mis en évidence sur milieu contenant l'IPTG - inducteur de l'opéron lactose - et le X-gal -groupe chromogène.

Les plasmides pUR222 qui ont inséré un fragment K88 en phase, apparaissent bleu sur milieu Xgal-IPTG. Une réaction antigénique contre les anticorps anti-K88 a été recherchée sur 1000 clones environ, présentant une coloration bleu clair à bleu foncé sur X-gal. Douze clones réagissent de manière très positive.

Le séquençage des inserts clonés dans les pUR222 révèle que les peptides

aa 69 à 92 ( = 23 aa) = FAT ....GAS

aa 83 à 122 ( = 39 aa) = IAF ....KVG

aa 147 à 160 ( = 13 aa) = LLS ....IFY

aa 217 à 229 ( = 12 aa) = YTD ....YAL

de K88ac sont antigéniques. Les segments contiennent donc des épitopes continus. Les trois premiers et les trois derniers acides aminés de ces fragments peptidiques sont donnés dans le code à une lettre. La numérotation des acides aminés est celle de la piline ac (référence est faite à la figure 3).

b1. Hydrophilicité

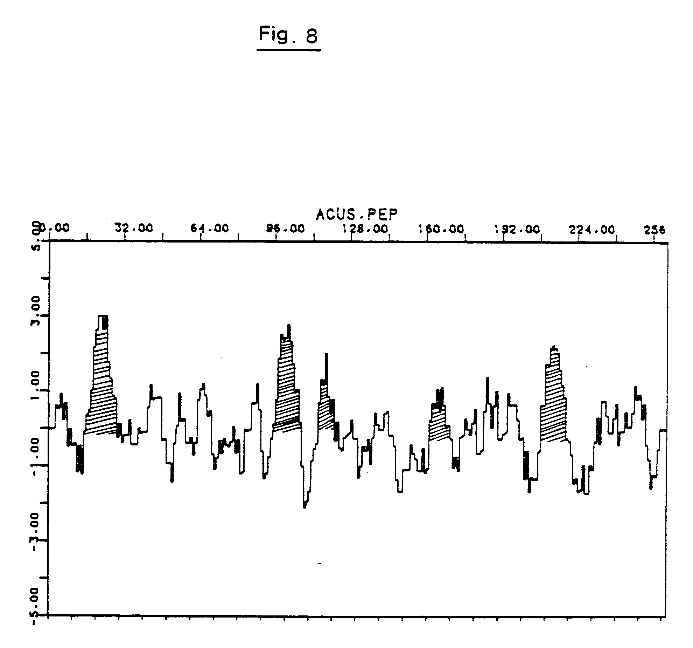

La figure 8 présente le profil d'hydrophilicité/hydrophobicité de la sous-unité K88ac, d'après l'algorithme de Doolittle. (Kyte et Doolittle, 1982, Mol. Biol., 157, 105-132).

Les parties hydrophiles de la molécule sont hachurées; chacune constitue une région exposée et un site antigénique potentiels.

Les peptides suivants ont été retenus

aa 17-29

aa 96-107 et aa 115-123

aa 162-170

aa 209-219 et aa 235-253

Ils sont particulièrement hydrophiles et de grande taille.

Pour la sous-unité K88ad, ce sont les régions suivantes qui ont été retenues :

aa 16 à 29

aa 87 à 106

aa 114 à 122

aa 162 à 175

aa 211 à 222 et 245 à 255.

b2. Présence de résidus proline

Les acides aminés prolines sont susceptibles de former des angles dans les protéines et d'exposer ainsi les régions voisines. Des prolines sont présentes aux aa 59, 72, 81, 98, 113, 164 et 254 de la piline ac. La présence de proline aux positions 98, 113, 164 et 254 qui sont situées à proximité immédiate ou dans des zones hydrophiles, est une indication supplémentaire favorable à la présence d'un épitope.

La sous-unité K88ad contient 8 résidus prolines, (aa 59, 72, 81, 98, 112, 163, 210 et 256). Ceux situés aux positions 98, 112, 163, 210 et 256 peuvent être mis en relation avec des régions hydrophiles.

b3 - Structure secondaire

La prédiction de structure secondaire de la piline K88ac, d'après l'algorithme de Chou et Fasman, montre que les régions hydrophiles retenues sont associées à des coudes dans la protéine.

5. Comparaison des sérotypes

K88 existe sous la forme de trois sérotypes : K88ab, ac et ad. Cette nomenclature signifie que ces sérotypes possèdent des antigènes communs "a" et distincts "b", "c" et "d". Chaque sérotype possède en outre des "variants".

Nous comparons a) les séquences et b) les profils d'hydrophilicité de toutes ces sous-unités.

a) Comparaison des séquences

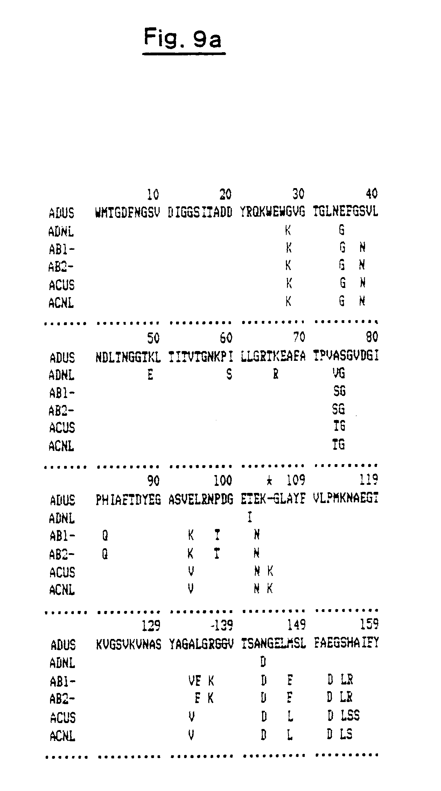

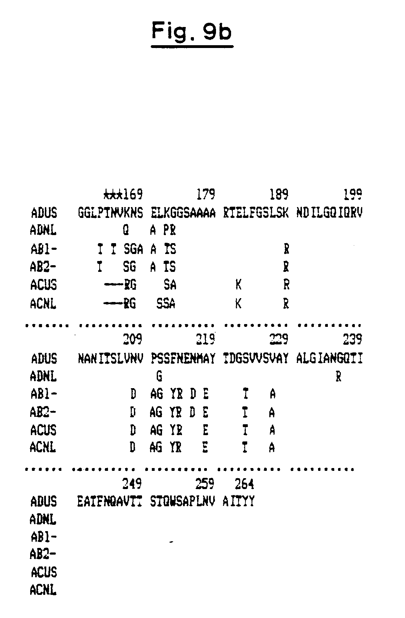

La figure 9 donne la séquence en acides aminés des variants ab (Dyker et al, 1985, Infection and Immunity, 50, 279-283), des variants ac (Josephsen et al, 1984, FEMS Microbiol. Letters, 25, 301-306) et celui analysé dans ce travail et d es variants ad (Gaastra et al, 1983, FEMS Microbiol. Letters, 18, 177-183). Les différences entre acides aminés sont seules reprises.

La relation entre les sites antigéniques et les séquences des trois sérotypes et leurs variants se base sur des postulats :

(1) - les sites antigéniques "a", qui sont communs aux 3 sérotypes et à leurs variants sont localisés dans des séquences invariantes. La figure 9 montre que les régions délimitées par les acides aminés 1 à 27, 38 à 48, 50 à 59, 83 à 93, 105 à 131, 190 à 207, et 237 à 264 sont constantes dans toutes les pilines.

(2) - les sérotypes b, c et d doivent se situer dans des régions variables entre les 3 sérotypes mais constantes entre leurs variants. Les régions 147 à 155 (148 à 151 pour ac) et 213 à 227 (211 à 225 pour ac) sont les deux seules régions qui répondent à ces deux contraintes.

En dehors de ces régions perturbées, seule la mutation ponctuelle à l'acide aminé 94 est variable entre les sérotypes b, c et d et communs à leurs variants.

(3) - les séquences 94 à 105, 133 à 136, et 163 à 173 diffèrent entre les variants et les sérotypes. Ceci est particulièrement frappant pour la séquence 163-173 dont la composition et la longueur sont différentes. D'un point de vue antigénique, ces séquences pourraient coïncider avec des antigènes variables, qui distingueraient les variants entre eux. La séquence 163-173 (cf. Fig. 9) est particulièrement variable dans ce sens.

b. Comparaison des profils d'hydrophilicité

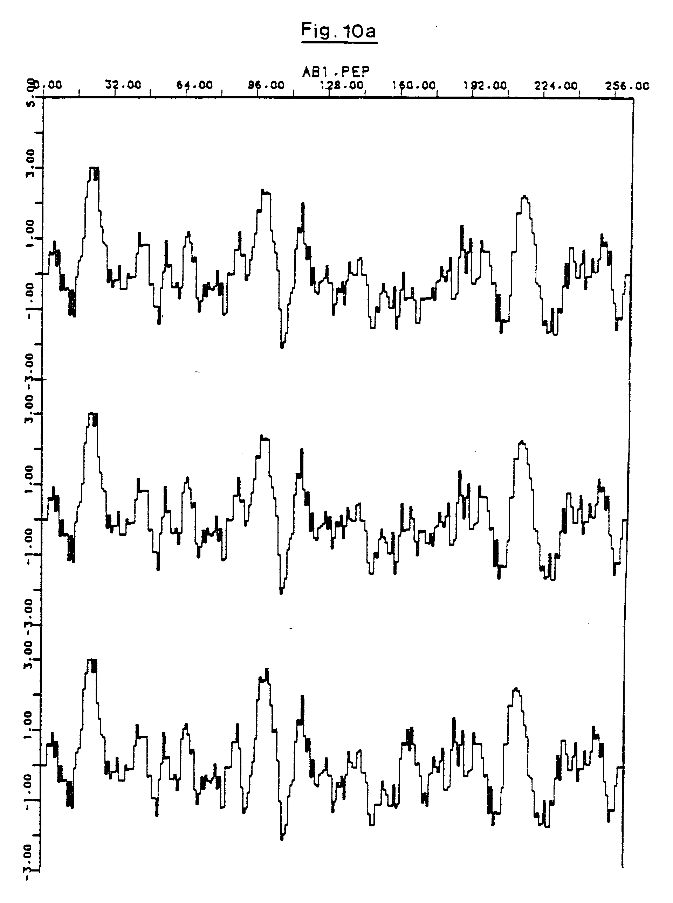

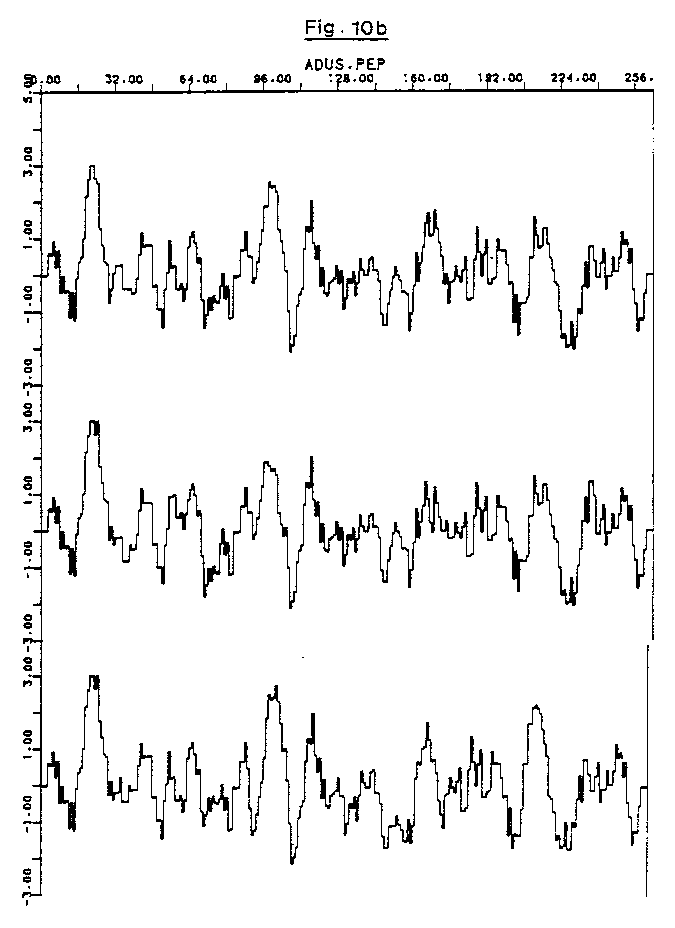

Les variations en acides aminés peuvent modifier les propriétés chimiques locales des séquences et en particulier l'hydrophilicité/hydrophobicité de la molécule. La figure 10 compare les profils des six sous-unités dont la composition est donnée à la figure 9, selon l'algorithme de Doolittle.

Les indices d'hydrophilicité des séquences hydrophiles indexées au point 4b permettent de déduire que :
- le fragment limité par les aa 17 à 29 est commun à toutes les sous-unités et nettement hydrophile;
- les fragments 96-107 et 115-123 sont également très hydrophiles; dans tous les pili;
- par contre, les séquences 162-175 et 211-222 ont des indices d'hydrophilicité variable. Ainsi, pour le sérotype ab, le peptide 162-175 n'est pas hydrophile, alors que le segment 211-222 est très hydrophile.

Au contraire, dans le sérotype ad, c'est l'inverse qui est observé : 162-175 est hydrophile, et 211-222 l'est moins (d'après Doolittle) sinon pas (d'après Hoop & Wood). Le sérotype ac présente une position intermédiaire.

c. Conclusions des points 3 et 4

Le tableau 2 reprend la position des peptides antigéniques, hydrophiles, invariables, et variables de la sous-unité K88ac, avec, pour le dernier point, la distinction entre la variabilité entre sérotypes et entre variants.

La dernière colonne est la sélection des peptides à la fois antigéniques et variables, qui sont au nombre de trois.

1. peptide 94 à 107.

Cette séquence reste douteuse du point de vue antigénique. En effet, les peptides antigéniques 69-92 et 83-122 pourraient contenir un épitope variable (94-105), ou commun, appartenant au sérotype a, (105-133 ou 84-90 peu hydrophile).

2. peptide 147-170

Le peptide 147-160 est antigénique. Il correspond à une séquence variable entre sérotype et pourrait donc correspondre au sérotype C. D'autre part, la séquence voisine 162-170 est extrêmement variable, en composition, longueur et hydrophilicité.

3. peptide 206-229

La séquence 206-225 diffère entre sérotypes et variants et son hydrophilicité est variable. Elle contient un épitope continu entre les acides aminés 217-229.

Ces fragments contenant des séquences antigéniques propres au pilus, sont susceptibles de modifications plus ou moins importantes qui change e caractère antigénique de la molécule sans en altérer les propriétés d'excrétion et de polymérisation.

Tableau 2

| Séquences antigéniques (ac) | Séquences hydrophiles (ac) | Séquences invariables (ac) | Séquences variables entre sérotypes | Séquences variables entre variants | Séquences antigéniques + variables (ac) |
|---|---|---|---|---|---|
| | 17-29 | 1-27 | | | |
| | 39-48 | 38-48 | | | |
| | 55-58 | 50-59 | | | |
| 69-92 | 84-90 | 83-93 | | | |
| | 96-107 | | | 94-105 | 94-107 ?? |
| 83-122 | 115-123 | 105-133 | | 134-136 | |
| 147-160 | 162-170 | | 148-151 | 162-170 | 147-170 |
| | 185-201 | 188-205 | | | |
| | 209-219 | | | 206-211 | 206-229 |
| 217-229 | 235-253 | 226-262 | 211-225 | | |

Tableau : Position des séquences antigéniques, hydrophiles, invariables et variables dans la sous-unité de K88ac et enfin des séquences à la fois antigéniques, hydrophiles et variables.

## 6. Choix d'un épitope

L'épitope choisi pour cet exemple est un épitope du virus de l'Influenza (Flu1). Il fait partie d'une région antigénique du virus, à la jonction entre l'hémagglutine HA1 et HA2.

La séquence peptidique

$$N'-P \quad E \quad K \quad \overset{5}{Q} \quad T \quad R \quad G \quad I \quad \overset{10}{F} \quad G \quad A$$

est immunogène lorsqu'elle est injectée à un animal sous forme d'un peptide synthétique (Brevet PCT/US83/01291, Scripps Clinic and Research Foundation).

Cette séquence existe dans la souche Vic[3] 75 (sérotype des virus de l'Influenza).

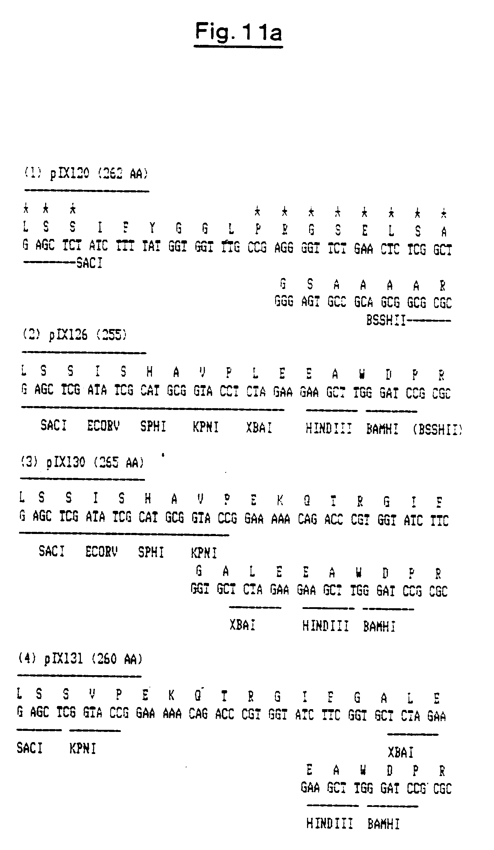

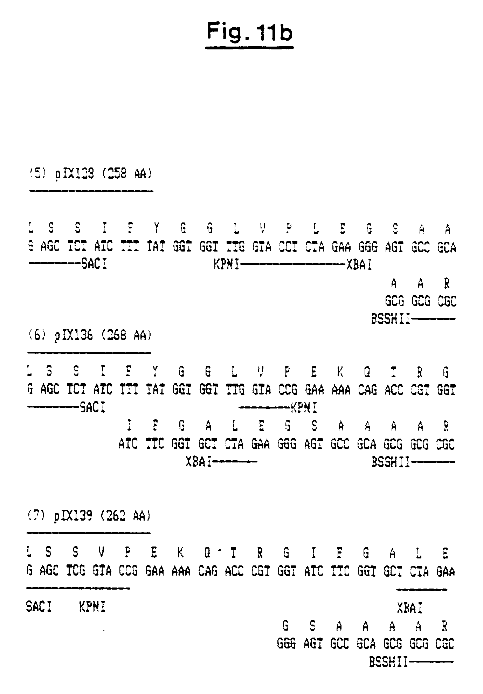

L'ADN codant pour cet épitope est obtenu par synthèse chimique. Le choix des codons tient compte de l'usage préférentiel des codons par E. coli. La séquence est présentée à la figure 11.

## 7. Clonage de l'ADN et expression du pili recombinant

La possibilité de modifier le gène de la sous-unité dépend de la présence de sites de restriction. Les 2 régions définies ci-avant : de aa 147 à 170 (ac) ou 146 à 173 (ad) est notée ci-après S1 et 206 à 229 (ac) équivalent à 208 à 231 dans les sérotypes ab et ad est notée S2.

En se reportant aux séquences nucléotidiques des sous-unités ac (fig. 3) et ad (fig. 5), il apparaît que la région S1 est clivée par l'enzyme SacI dans le sérotype ac et EcoR1 dans le type ad. SacI clive dans la région antigénique 147-160, et EcoR1 restreint dans le segment très variable 163-173 de ad. Deux sites intéressants, BgII et BssHII, clivent non loin de S1, aux aa 175 et 178 pour ac et 177 et 180 pour ad.

La région S2 est accessible dans le gène K88ad grâce aux sites de restriction HindII/HpaI (aa 207/208) et XhoII (222 et 223). HindII est situé au début de la séquence variable; XhoII clive une séquence antigénique.

Les manipulations suivantes sont réalisées pour S1 et S2 successivement.

### a. Manipulation de S1

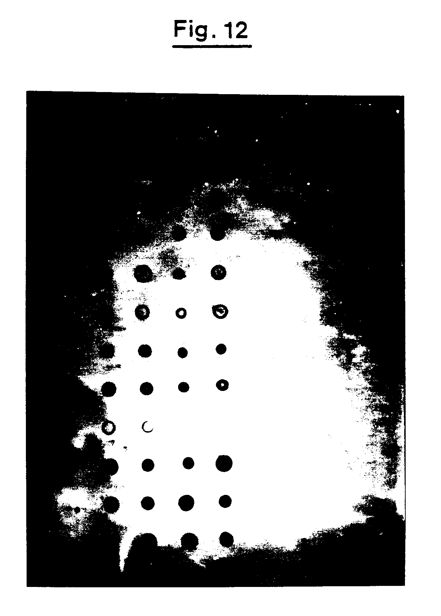

Les modifications de la région S1 sont données à la Figure 11. La production de pili par les souches recombinantes est tout d'abord testée par un test immunologique sur colonie. La figure 12 montre la réaction immunologique obtenue pour les différentes constructions décrites ci-dessous.

### 1. Utilisation d'un polylinker

Clonage

Dans le but de tester la versatilité de la région S1, le fragment SacI-BssHII du gène de la piline K88ac porté par le pIX120 a été remplacé par le linker synthétique du M13tg130, cloné dans un vecteur pUC. La construction du pIX126 est réalisée en deux étapes : le clonage de la partie 5′ du gène de la sous-unité en amont du site SacI du pUC 130 tout d'abord est le résultat de la ligation des fragments PvuI-SacI du pUCtg 130, et SacI -Pvu I du pIX126. Le plasmide est ensuite restreint par BamH1, rendu blunt ended par la polymérase-fragment Klenow, puis restreint par PvuI. La partie C terminal du gène de la sous-unité est isolée sur le fragment BssHII/Klenow/PvuI du pIX120. Le gène de la sous-unité est reconstitué par la ligation des fragments pVuI-BamH1 et BssHII - PvuI pour générer le pIX126. La séquence de ce plasmide a été vérifiée. La séquence du linker est donnée à la figure 11, qui montre le fragment SacI-BamH1 introduit en lieu et place de S1, et sa traduction en acides aminés dans la sous-unité. Notons que la taille de la sous-unité est réduite de 262 aa à 255 acides aminés.

Expression

Le pIX126 est ensuite introduit, par transformation dans une souche contenant le système de complémentation (pIX211) et la présence de pili est recherchée par
- agglutination : une faible agglutination est observée
- test immunologique sur bactérie : l'anticorps polyclonal réagit positivement avec les bactéries, mais de manière moins intense que sur la souche témoin. P ar contre, des anticorps monoclonaux spécifiques K88ac ne reconnaissent pas les pili recombinants. Ces anticorps sont spécifiques au sérotype commun "a" parce qu'ils reconnaissent à la fois K88ab, ac et ad, et en particulier la protéine native, non dénaturée. L'absence de réaction avec les pilines produites par pIX126 laissait présager

17

des modifications de structure des pili.

- purification de pili et gel d'acrylamide. La présence de pili a été recherchée par rasage d'une culture liquide de la bactérie contenant pIX126 et pIX211, dans les mêmes conditions que la bactérie témoin porteuse de pIX120 et pIX211. Les préparations sont analysées sur gel d'acrylamide. Aucune sous-unité piline n'apparaît dans la préparation de la bactérie recombinante.
- un ELISA sandwich détecte de l'ordre de 0,1 micro g de pili par ml de rasat brut d'une culture de pIX126.

Les conclusions de cette première modification pourraient être que la piline modifiée est bien excrétée de la bactérie mais n'est pas assemblée en de longs filaments.

## 2. Clonage de l'épitope dans le polylinker

L'addition de l'épitope de l'Influenza décrit au point 6 dans le polylinker augmente la taille de la sous-unité de 10 acides aminés.

### Clonage

L'ADN synthétique codant pour l'épitope de l'Influenza possède des extrémités cohésives KpnI - XbaI. Il a été tout d'abord cloné, en phase, dans le polylinker du pUC18; ce plasmide induit la production d'une beta-galactosidase hybride contenant l'épitope viral.

L'ADN synthétique est également cloné dans le pIX126 pour générer le pIX130.

### Expression

Le pIX130 introduit dans la souche de complémentation induit la fabrication et l'excrétion de sous-unités reconnues par les polyclonaux, mais non par les monoclonaux anti K88. Aucune structure identifiable aux pili n'est cependant extraite d'une culture liquide de cette bactérie, comme le montre les résultats des dosages par Elisa (cf. Tableau 3).

### Clonage

Le site SacI a les mêmes phases de lecture dans pUC18 et le pIX126 (AGC code pour une sérine). Dans le pIX126, la séquence SacI-EcoRV-SphI-KpnI a été remplacée par la séquence SacI-KpnI du PUC18. Le plasmide qui en résulte, noté pIX131, contient l'épitope de l'influenza et code pour une sous-unité de 260 résidus.

### Expression

Le pIX131 introduit dans la souche de complémentation est reconnu par les anticorps polyclonaux anti K88 de la même manière que le pilus produit par la souche contenant le pIX126. Cette sous-unité semble être excrétée mais mal sinon pas assemblée.

Une très faible quantité d'antigènes pili (de l'ordre de 10 ng par ml) est détectée dans les rasats bruts (cf. Tableau 3).

## 3 - Utilisation d'un adaptateur synthétique

La comparaison des séquences en acides aminés des diverses pilines, présentée à la figure 9, montre que, schématiquement, la région S1 est constituée de 3 séquences : six aa constants (I, F, Y, G, G, L), 8 aa variables (11 dans les sérotypes ad et ab), puis une large région invariable, qui couvre le site BssHII.

Ces deux régions invariables pourraient jouer un rôle dans la production du pili et leur absence expliquerait la perte de production de pili par les recombinants pIX126 et ses dérivés pIX130 et 131.

Nous avons dès lors réintroduit ces deux régions constantes dans la séquence S1 de la manière suivante :

### Clonage

Un adaptateur dont les extrémités sont cohésives avec les sites SacI et BssHII et codant pour les acides aminés invariables de la région S1 a été synthétisé. La région variable est remplacée par les sites KpnI et XbaI, qui permettront le clonage futur de l'épitope.

La séquence de cet adaptateur noté Ep34 apparaît à la fig. 11. Cet ADN a été cloné dans les sites SacI-BssHII du pIX120 suivant la technique classique : restriction SacI-BssH2 du pIX120, et ligation en présence d'un excès d'adaptateur. Ce plasmide est le pIX128.

Expression

La sous-unité synthétisée par le pIX128 est constituée de 258 acides aminés.

Les tests immunologiques sur colonies des bactéries d'expression contenant le pIX128 sont clairement positifs, que les pili soient recherchés grâce à des anticorps polyclonaux ou monoclonaux.

La concentration en pili recombinants dans un rasat brut est de 3 à 6 micro g/ml. (Elisa sandwich dans lequel les anticorps adsorbés sont des polyclonaux, cf. tableau 3), comparés aux 16-20 micro g de pili sauvages/ml. Les pili sont donc toujours assemblés, mais sont en moindre quantité, de l'ordre de 5 fois moins que des pili non recombinants.

La sous-unité recombinante du pIX128 a été mise en évidence sur un gel PAGE et sur un immunoblot.

4. Addition de l'épitope dans l'adaptateur synthétique

Le DNA synthétique qui code pour un épitope de l'Influenza possède des extrémités compatibles avec les séquences Kpn1 et XbaI de l'adaptateur cloné dans le pIX128. L'étape suivante comporte donc le clonage de cet épitope en phase dans le pIX128, générant le pIX136.

Clonage

Le pIX128 est restreint par les endonucléases de restriction KpnI et XbaI et mis en présence d'un excès du DNA synthétique codant pour l'épitope viral. Après ligation, les plasmides sont introduits dans EC294 par transformation.

Expression

Le plasmide pIX136 induit dans la souche EC294 (pIX211) la production de pili reconnus par des anticorps anti-K88 poly-et monoclonaux (cf. figure 12).

L'Elisa révèle la présence d'antigènes pili dans les rasats bruts. La concentration en antigène est cependant assez faible, de l'ordre de 1-3 micro g/ml.

En appliquant 1 micro g de pili sur un gel PAGE et sur immunoblot, on met en évidence une sous-unité d'un poids moléculaire légèrement supérieur à celui de la sous-unité native.

5. Réduction de la taille de la piline recombinante

La sous-unité produite par le pIX136 a une taille équivalente à 268 acides aminés (aa), soit 6 acides aminés de plus que la sous-unité naturelle ac. Nous avons délété le fragment d'ADN situé entre les sites SacI-KpnI du pIX136 (cf. figure 11.b). Ce DNA code pour 6 acides aminés invariables, situés du côté N-terminal de l'épitope du virus. Il est intéressant de noter que, ce faisant, l'épitope cloné est rapproché du site SacI qui est compris dans une séquence traduite antigénique : la séquence piptidique LLSIFY est un épitope du pilus.

Clonage

Le gène pilus du pIX139 possède la partie 5′ du pIX131, jusqu'au site Xba - y compris donc l'épitope de l'Influenza -, et la partie 3′, à partir du site XbaI du pIX128. Les 6 premiers acides aminés (aa) constants de la région S1 ont donc été délétés.

Expression

La bactérie qui contient à la fois le système de complémentation et le pIX139 est faiblement reconnue par les anticorps anti-pili K88 polyclonaux mais n'est pas reconnu par les monoclonaux.

L'Elisa d'un rasat brut de cette souche détecte très peu d'antigènes, environ 10 à 20 ng/ml, ce qui est à la limite de détection de cette méthode dans nos conditions. Aucun antigène n'est décelé lorsque des monoclonaux sont utilisés pour l'Elisa (cf. Tableau 3).

L'immunoblot d'un large extrait du rasat brut laisse apparaître une bande peu intense dont la migration correspond à celle d'une sous-unité de pilus.

## 6. Conclusions pour S1

Les conclusions suivantes ressortent des différentes manipulations de S1.
- La région variable de 8 acides aminés de S1 peut être délétée de la sous-unité sans supprimer sa propriété d'excrétion de d 'assemblage.
- Elle peut être remplacée par une autre séquence, totalement inédite et plus longue de 6 acides aminés.
- La séquence constante située en amont de la région variable est essentielle à la fabrication du pilus.
- La quantité de pili que l'on peut extraire des deux souches recombinantes est faible; en prenant comme référence la quantité de pili purifiés d'une souche sauvage, elle chute à 20 % pour la souche contenant le pIX128 et à 6 % pour celle porteuse du pIX136.

## b. Manipulation de la région S2

Rappelons que la région S2 définie au point 4 se situe entre les acides aminés 208 et 231 de la piline K88ad. Ce qui suit décrit les diverses manipulations de cette région.

## 1. S2 est interchangeable entre sérotypes

La région S2 a une composition et une hydrophilicité différentes entre les sérotypes ac et ad. Une première manipulation a consisté à remplacer la région S2 de ad par celle de ac et, inversément, de cloner S2 de ad dans le gène de la piline ac.

## Clonage

Les plasmides pIX120 et pAD120 (cf. figures 4 et 6 respectivement) sont clivés par BglI. Chaque plasmide contient deux sites BglI : l'une est situé dans le gène de résistance à l'ampicilline, l'autre dans le gène de la piline, 100 nucléotides en amont de la région S2. Le fragment du pIX120 porteur du début du gène de la piline ac est ligaturé au fragment du pAD120 porteur de l'extrémité 3′ du gène de la piline ad. Cette ligation reconstitue un gène de résistance à l'ampicilline, et une sous-unité piline hybride ac-ad. Ce plasmide est le pIX134. De la même manière, les deux autres fragments sont ligaturés pour reconstituer cette fois une piline ad-ac, ce second plasmide est le pIX135. Ces plasmides sont aisément distingués des molécules parentales pIX120 et pAD120 par restriction EcoR1 notamment.

## Expression

Introduits dans la souche EC294 (pIX211), ces deux plasmides induisent l'excrétion de pili que l'on peut extraire. A noter que la piline codée par le pIX134 (ac-ad) a la migration électrophorétique de la piline ac, alors que la sous-unité déterminée par le plasmide pIX135 (ad-ac) migre plus lentement sur gel PAGE-SDS, comme la sous-unité du type ad.

## 2. Remplacement de la S2 par un adaptateur synthétique

Le remplacement de la région S2 par un adaptateur synthétique permet d'introduire des sites de restriction multiple dans la région S2, et de tester sa versatilité.

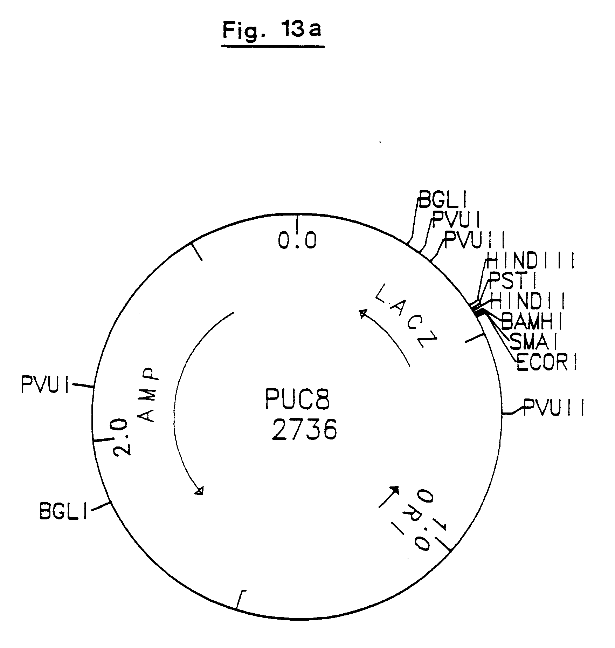

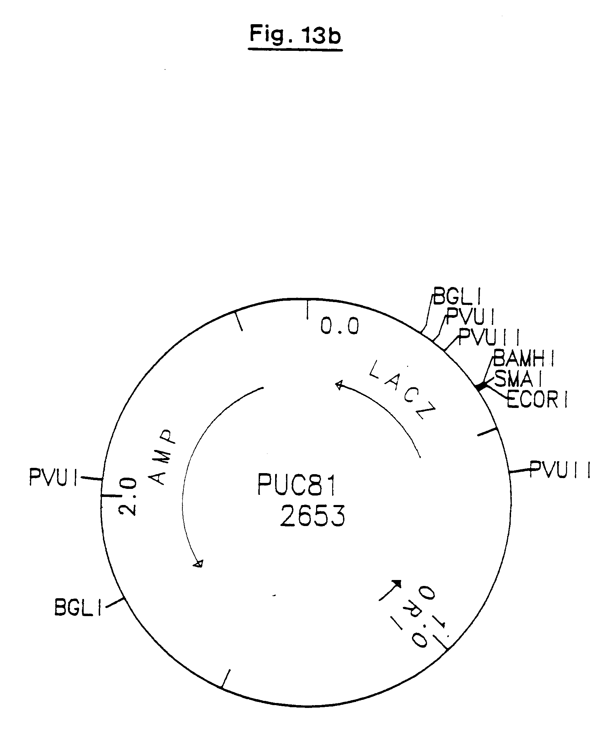

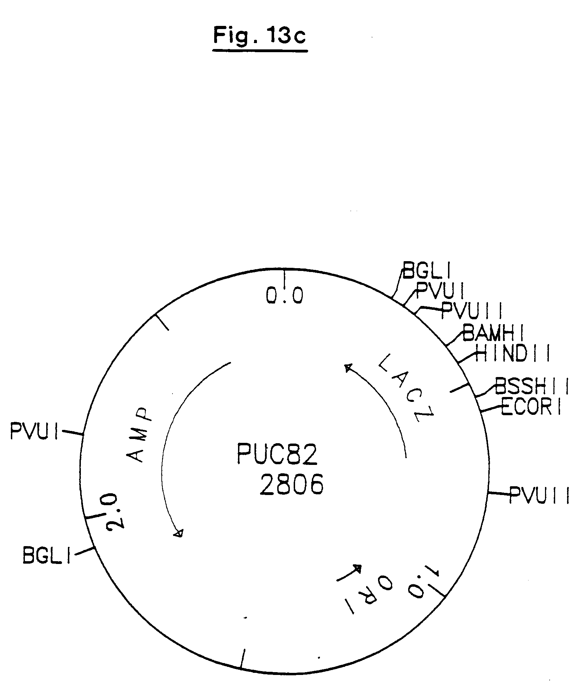

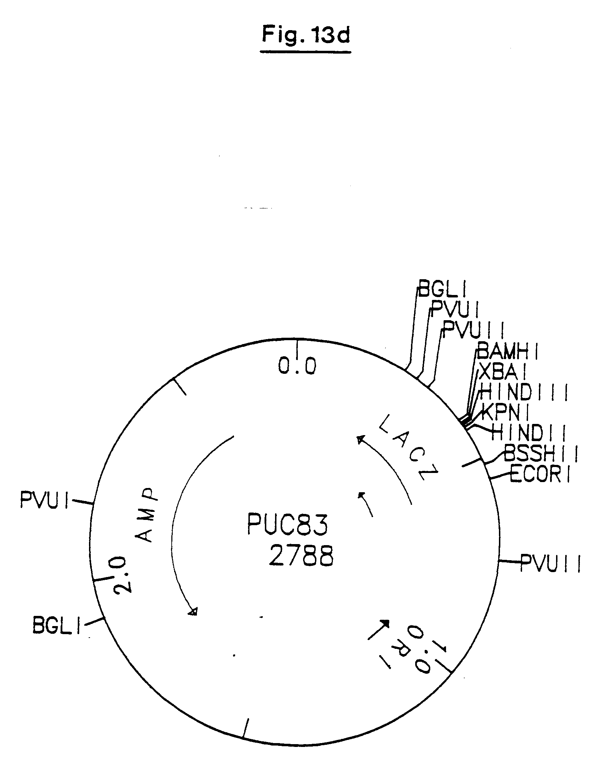

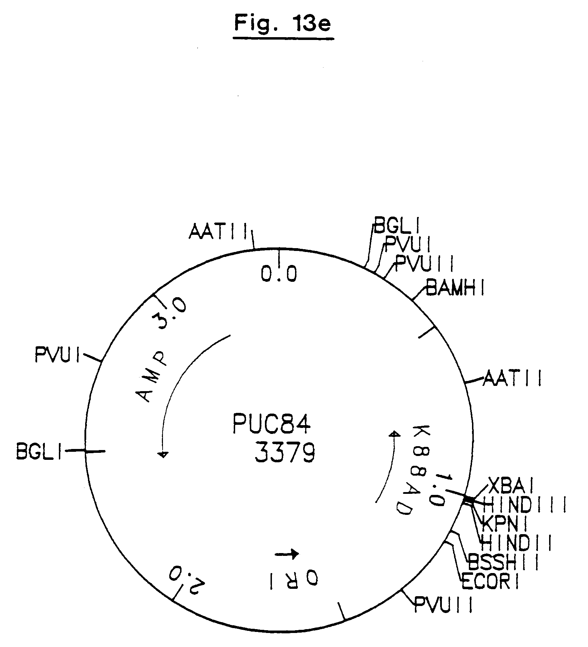

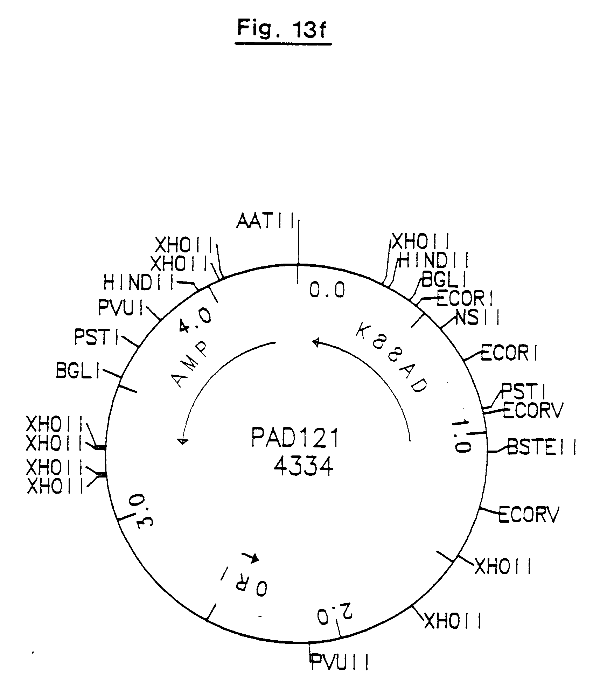

Comme souligné auparavant, la région du gène de la piline ad contenant S2 est acessible aux manipulations génétiques via les sites HindII et XhoII. Pas directement cependant car il existe 9 sites de restriction XhoII dans le pAD120. Le remplacement du fragment HindII-XhoII du gène piline par un DNA synthétique ne pouvait donc être réalisé dans le pAD120. Comme XhoII possède des extrémités cohésives avec BamH1, une stratégie de clonage a été adoptée dans laquelle le site multiple XhoII est remplacé par un site unique BamH1. Le clonage fait intervenir quatre intermédiaires, dérivés du vecteur pUC8. Ces intermédiaires sont présentés aux figures 13.

## Clonage

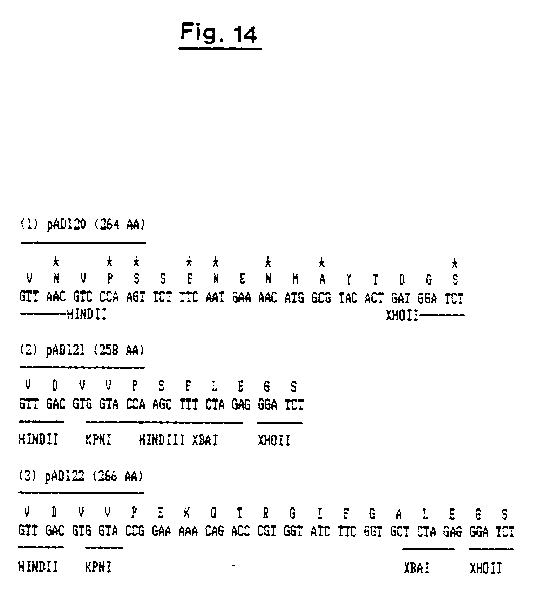

- Dans le but d'utiliser le site HindII du gène de structure de la piline, le site HindII du linker du pUC8 a été préalablement délété, générant le pUC81. Cette délétion est réalisée par restriction du pUC8 par les enzymes HindII et HindIII, traitement à la polymérase - fragment klenow - de E. coli et ligation. Cette délétion maintient le caractère Lac + (fig. 13.a et 13.b).
- pUC 82 est le second intermédiaire construit par le clonage en phase du fragment EcoR1-XhoII du gène ad dans les sites EcoR1-BamH1 du PuC81. Cette insertion entraîne la perte du site SmaI du linker pUC81, et maintient le caractère Lac +. Le site multiple XhoII (GGATCT) est maintenant remplacé par un site unique BamH1 (GGATCC) (Fig. 13c).
- L'adaptateur synthétique EP56, dont la séquence est donnée à la figure 14, est cloné en phase dans les sites HindII-BamH1 du pUC82. Ce pUC83 contient donc un fragment du gène piline ad, avec une nouvelle région S2 (Fig. 13d). Il faut maintenant reconstituer le gène complet, ce qui est réalisé dans les deux dernières étapes.
- Clonage du fragment XhoII-XhoII du pAD120, fragment porteur de l'extrémité 3′ du gène piline, en phase dans le site BamH1 du pUC83 (Fig. 13e). Ce clonage confère le caractère Lac⁻ à la souche JM103. Les deux insertions possibles du fragment sont distinguées par restriction AatII.
- Enfin le remplacement du fragment BssHII-AatII du pAD120 par son homologue du pUC84 permet de reconstituer un gène piline complet, recombinant. Ce plasmide est le pAD121. Sa carte de restriction apparaît à la figure 13f.

Expression

La souche d'expression EC294 (pIX211)(pAD121) donne une réponse immunologique intense (cf. fig. 12) et synthétise des pili que l'on peut extraire par les techniques classiques.

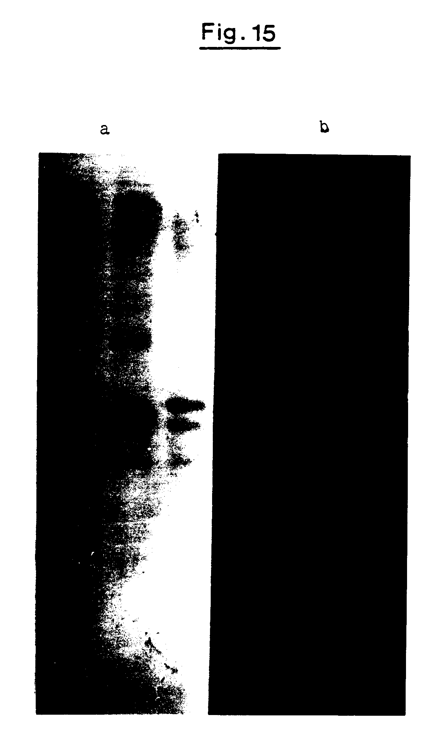

La figure 15 montre une photographie d'un gel PAGE-SDS d'un rasat brut des cultures productrices de pili sauvages et recombinants. La piline recombinante codée par le pAD121 a une mobilité électrophorétique un peu plus grande que la piline ad sauvage. Ceci est attendu puisque la modification de S2 réduit la taille de la sous-unité de 264 acides aminés à 258 résidus.

Analysée par Western Blot, cette sous-unité est parfaitement reconnue par les anticorps polyclonaux anti-K88, comme le montre la figure 15.b.

La concentration en antigène d'un rasat brut de la souche EC294 (pIX211)(pAD121) est d'environ 25 micro g/ml, soit identique à celle d'un rasat d'une souche ad non recombinante. Contrairement à ce que nous avons observé pour S1 (cf. l'équivalent du pAD121, le pIX128) la délétion de la région variable ne diminue pas la quantité de pili recombinants que l'on peut purifier.

3 - Introduction d'un antigène dans S2

La région S2 du gène piline recombinant offre de nombreuses possibilités de clonage d'un fragment de DNA hétérologue grâce à la présence des sites de restriction unique kPn1, HindIII et XbaI.

Dans cet exemple, le DNA synthétique codant pour l'épitope Flu1 de l'Influenza a été cloné dans les sites KpnI et XbaI.

Clonage

Le vecteur pAD121, restreint par KpnI et XbaI est ligaturé en présence d'un exès du DNA synthétique Ep12. La séquence de cet ADN est donnée à la figure 14. Le plasmide recombinant est le pAD122.

Expression

Comme le prouve le test immunologique sur colonie (fig. 12), la souche EC294 (pIX211) (pAD122) réagit aux anticorps anti-K88ac. Cette souche excrète des pili recombinants que l'on peut extraire, comme le montrent les gels PAGE et immunoblot des figures 15a et b.

Nous avons pu estimer la quantité de pili présente dans un rasat brut à 7 micro g/ml, soit environ 3,5 fois moins que dans les rasats des pAD120 et 121 (cf. Tableau 3).

4. Conclusions pour S2

La région S2 apparaît versatile et tolérante, et ceci à la fois pour sa longueur globale et pour sa composition. En effet, Les résultats montrent qu'une délétion de 6 résidus (pAD121) ou une insertion finale de 2 acides aminés (aa) (pAD122) maintiennent l'assemblage du pili. On peut remarquer que la région S2 du pAD121 contient des acides aminés (aa) identiques ou chimiquement proche de ceux présents dans le pAD120 (cf. fig. 14). C'est le cas pour la proline (P), phenylalanine (F), valine (V), sérine (S) et l'acide glutamique (E) qui sont identiques, ou l'acide aspartique (D), remplacé par l'acide glutamique (E). L'insertion de l'épitope viral dans le pAD122 change par contre complètement la composition de S2 et le maintien de la synthèse de pili prouve la grande tolérance de cette séquence. Il faut cepend ant pondérer ce résultat en rappelant que la quantité de pili purifiables est diminuée d'un facteur 3.

c. Assemblage en pili de pilines modifiées

1. Test sur colonie

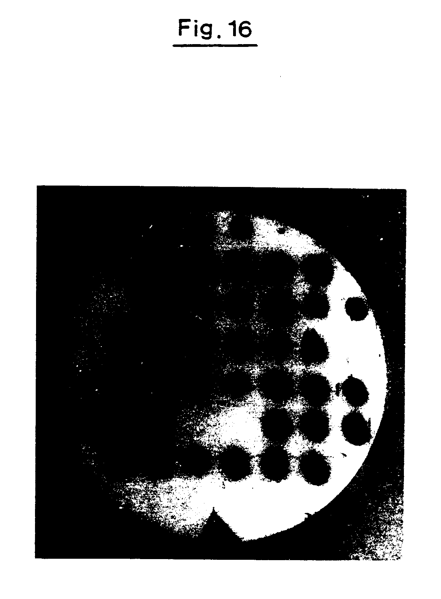

La figure 16 qui est la photographie d'un filtre, montre le résultat d'un test immunologique sur colonie bactérienne qui utilise un sérum polyclonal anti-K88 comme moyen de détection. Les colonies intéressantes sont celles qui hébergent (i) les plasmides non modifiés (pIX120, pAD120), qui servent de contrôles positifs, (ii) les plasmides "vecteurs", contenant un linker en lieu et place des séquences variables S1 et S2 (pIX128, pAD121), et enfin, (iii) les plasmides qui codent pour les pilines qui expriment un épitope du virus de l'Influenza (pIX136, pAD122). Leurs positions sur le filtre sont, dans cet ordre :

```
non modifié : pIX120, ligne 4, clones 1, 2 et 3
              pAD120, ligne 1, clones 1, 2 et 3
vecteur     : pIX128, ligne 4, clones 4, 5 et 6
              pAD121, ligne 1, clones 4, 5 et 6
fusion      : pIX136, ligne 5, clones 1, 2 et 3
              pAD122, ligne 2, clones 1, 2 et 3
```

Deux contrôles négatifs sont présents :
- la souche E. coli ne contenant que les plasmides pIX211, porteur des gènes helpers, et pUR222. Elle ne contient par conséquent pas de gène piline. Elle est à la ligne 3, clones 4, 5 et 6 sur le filtre.
- la souche contenant le plasmide pACYC184, vecteur utilisé pour le clonage du pIX211, et le pIX120. Cette souche produit donc la sous-unité, mais est incapable de l'assembler en pili.

La réaction anticorps-antigènes observée sur ces contrôles négatifs est faible, et considérée comme du bruit de fond.

Les bactéries qui produisent les pili non modifiés (IX120 et AD120) sont par contre fortement reconnues par les anticorps, ainsi que toutes les bactéries qui expriment les pilines recombinantes (IX128 et 136, AD121 et 122). L'intensité des taches, qui peut refléter la quantité d'antigènes présents, est relativement importante pour chaque recombinant.

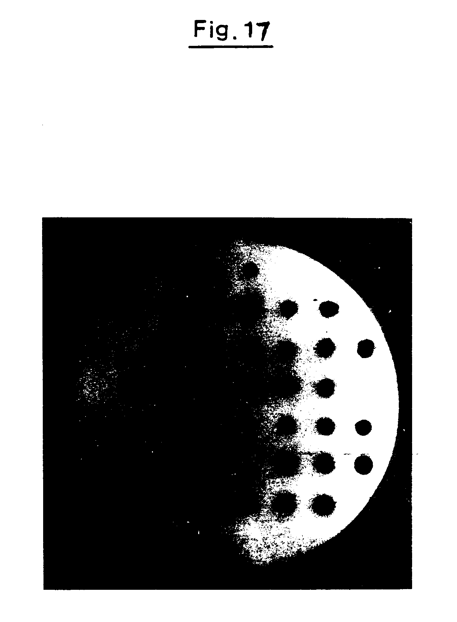

Le même test a été réalisé sur le fitre présenté à la figure 17, en utilisant cette fois un anticorps monoclonal (MAb) spécifique des pili K88 comme moyen de détection. Ce MAb a été choisi parce qu'il reconnaît le pili intact, mais très peu le pili dénaturé dissocié en piline. Dans la mesure où son épitope conformationnel est conservé, cet anticorps distingue le pili recombinant de la piline libre. Comme le montre la figure 17, les bactéries qui expriment la piline sauvage ac ou ad sont reconnues, ainsi que toutes les bactéries qui synthétisent les pilines recombinantes IX128 et 136, AD121 et 122. Aucun bruit de fond n'est détecté sur le contrôle négatif. Donc ces pilines sont effectivement associées en pili attachés à la surface de la bactérie. En admettant que l'affinité de l'anticorps soit la même pour chaque pili modifié, l'intensité de la réaction suggère une plus faible quantité d'antigènes sur les souches porteuses des plasmides pIX128 et pIX136, ainsi que sur les souches porteuses du plasmide pAD122.

2. Elisa

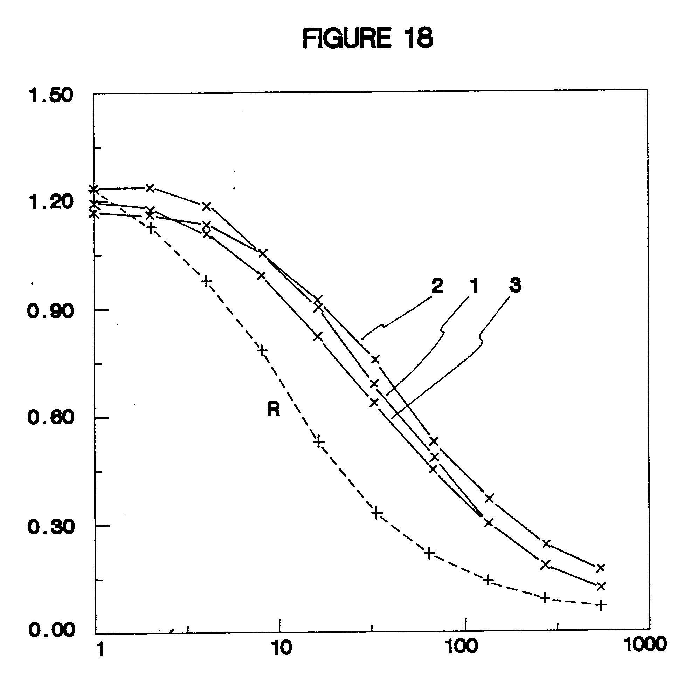

Des extractions de pili recombinants modifiés en S1 ou S2 ont été réalisées en leur concentration a été estimée par Elisa. Un exemple de dosage Elisa est présenté à la figure 18. Les rasats des souches recombinantes sont obtenus par traitement thermique comme décrit dans la partie "Méthodes", et sont adsorbés sur la plaque. Ils sont révélés par un anticorps monoclonal anti-K88 dilué de 2 en 2 sur la plaque. Les rasats analysés sur cette figure sont ceux extraits des souches qui expriment les gènes du pIX120, pIX128 et pIX136. Les concentrations en antigènes K88 sont estimées par rapport à une solution de pili de référence à 1 micro g/ml. Elles sont respectivement, tenant compte des facteurs de dilutions, de :
15 micro g/ml pour pIX116
8 micro g/ml pour pIX128
3 micro g/ml pour pIX136.

Ces valeurs sont proches de celles présentées au tableau 3.

Le même type d'expérience a confirmé les résultats obtenus pour les souches qui contiennent les plasmides pAD120, 121 et 122 (cf. tableau 3).

## 3. Microscopie électronique

La microscopie électronique met en évidence des pili qui ont les caractéristiques morphologiques du pili K88, autour des souches qui expriment les gènes pIX128, pAD121 et pAD122.

## 8. Antigénicité du peptide fusionné au pilus

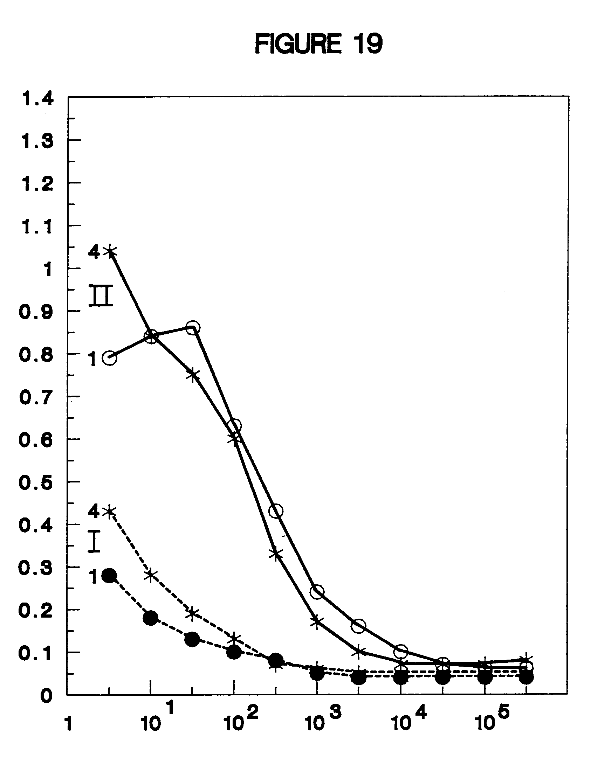

Un peptide synthétique ayant la séquence de l'épitope de l'Influenza (séquence appelée Flu1) est utilisé pour immuniser des souris. Les cellules de la rate des souris immunisées servent à fabriquer des hybridomes producteurs d'anticorps. L'analyse des anticorps permet de sélectionner des monoclonaux qui reconnaissent le peptide de synthèse avec une forte affinité et ne réagissent pas avec le pili K88. La réaction de ces monoclonaux contre des pili recombinants est ensuite analysée par Elisa. Des pili IX121 et IX136 ainsi que AD121 et AD122 sont adsorbés sur les plaques Elisa, puis incubés en présence de dilution d'anticorps monoclonaux anti-Flu1. Deux anticorps présentent une réaction plus importante sur les pili recombinants que sur les contrôles, comme illustré à la figure 19. Par contre, ce test réalisé en adsorbant des pili dénaturés à la chaleur ne montre pas de nette différence de réactivité entre les pili porteurs de la séquence Flu1 et les contrôles.

Ces expériences prouvent que (i) le peptide Flu1 fusionné aux pilines assemblées en pili est accessible aux anticorps monoclonaux et (ii) que l'antigénicité du peptide est amplifiée lorsqu'il est porté par une structure.

## 9. Immunogénicité du pili recombinant

15 mg de pili AD122 a été purifié par extraction mécanique d'une culture EC294 (pIX211)(pAD122). Les antigènes ont été concentrés par ultrafiltration et leur concentration et pureté ont été estimées par Elisa et gel PAGE. Ces antigènes ont servi à immuniser des rats, dans les conditions expérimentales suivantes : chaque rat a subi deux injections intra péritonéale, espacées de quinze jours, de deux ml d'émulsion contenant 1 ml d'adjuvant complet de Freund, et 1 ml d'une des solutions contenant
- du tampon de dilution
- 1 micro g de pili dans le tampon de dilution
- 3 micro g de pili dans le tampon de dilution
- 10 micro g de pili dans le tampon de dilution
- 30 micro g de pili dans le tampon de dilution
- 100 micro g de pili dans le tampon de dilution
- 300 micro g de pili dans le tampon de dilution

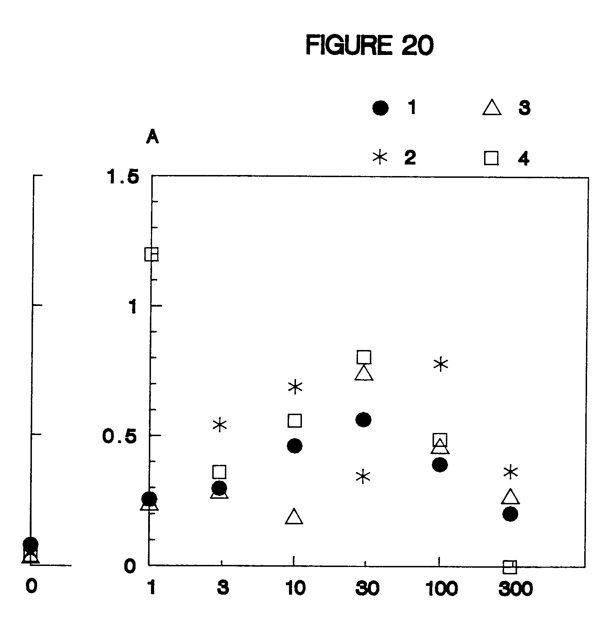

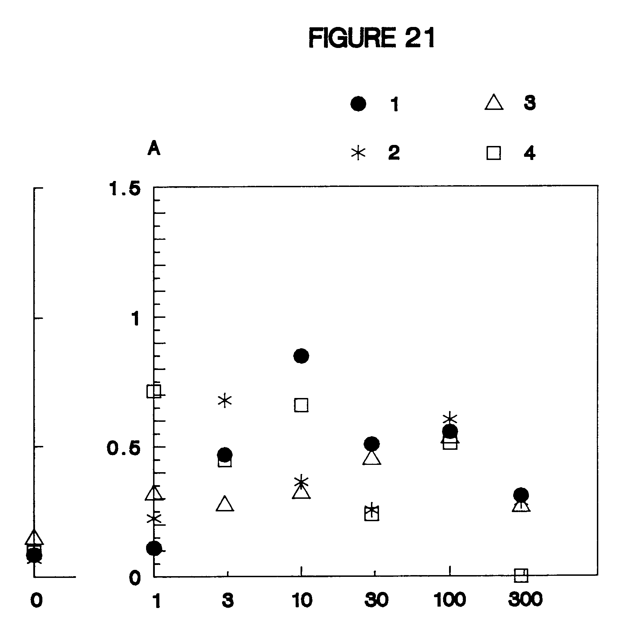

Sept groupes de quatre rats chacun ont ainsi été traités. Du sérum a été prélevé avant la première immunisation (sérum préimmun), avant la seconde injection, et quinze jours après celle-ci. Ils furent analysés par Elisa pour leur réaction sur le pili K88 et sur le peptide de synthèse conjugué à la BSA (bovine serum albumine). Aucun des sérums préimmuns de ces rats ne réagit contre les antigènes pili et Flu1. Quinze jours après la première injection par contre, il apparaît une réponse importante contre le pili, mais aucune réponse contre le peptide. Enfin, quinze jours après la seconde injection, la réponse contre le pilus se confirme, et une réaction importante contre le peptide apparaît. Ces derniers résultats sont illustrés aux figures 20 et 21, sur lesquelles sont reportées les réponses individuelles de chaque rat contre le pilus et le Flu1. Les quantités d'antigènes injectés sont en abscisse, et la valeur de la densité optique pour la dilution 10 du sérum est reprise en ordonnée. La figure 20 montre que l'optimum de la réponse contre le pilus s'obtient pour des injections de 3 à 100 micro g d'antigènes. La figure 21 prouve que les sérums contiennent des anticorps dirigés contre le peptide. Ici l'optimum de la réponse s'obtient pour des injections de 3 à 30 micro g d'antigènes.

Ce résultat prouve que l'immunisation avec un pili recombinant induit la sélection d'anticorps contre

EP 0 264 150 B1

l'épitope étranger qu'il comprend ou, en d'autres termes, que l'épitope étranger fusionné au pilus est rendu immunogénique. Ce peptide est présent en très faible quantité. En effet, l'épitope Flu1 est long de 12 acides aminés, soit à peine 4,5 % de la piline AD122 totale. En injectant 10 micro g d'antigènes, on immunise en réalité avec 0,45 micro g de peptide. L'intensité de la réponse humorale contre ce peptide suggère un sérieux effet d'amplification du pouvoir immunogénique du peptide par le pilus.

Tableau 3

| Nom du plasmide | Concentration en antigène.K88 (micro g/ml) | % |
|---|---|---|
| pIX116 | 16 | 100 |
| pIX126 | 0,1 | 0,6 |
| pIX130 | 0,01 | - |
| pIX131 | 0,01 | - |
| pIX128 | 3 | 18 |
| pIX136 | 1 | 6 |
| pIX139 | 0,01 | - |
| pAD120 | 25 | 100 |
| pAD121 | 25 | 100 |
| pAD122 | 7 | 28 |

Tableau 3 : Concentration en antigène K88 dans différents rasats bruts (purification à la chaleur) déterminée par Elisa Sandwich. L'anticorps adsorbé est un polyclonal anti-K88ac. La concentration en protéine est de 50-60 micro g/ml, déterminée par Coomassie. Ces valeurs sont la moyenne de trois mesures. Les références sont des pili purifiés des sérotypes ac pour la série pIX et du sérotype ad pour la série des pAD, amené à la concentration de 1 micro g/ml.

Une souche d'Escherichia coli contenant un premier plasmide pIX211 et un second plasmide pIX136 a été déposée à la NCIB sous la référence EC294 (pIX211)(pIX136) - (NCIB numéro 1234 9). Ce dépôt a été réalisé conformément au Traité de Budapest. Une seconde souche d'Escherichia coli contenant un premier plasmide pIX211 et un second plasmide pAD122 a été déposée à la NCIB sous la référence EC294 (pIX211)(pAD122) - (NCIB numéro 12347). Ce dépôt a également été réalisé conformément au Traité de Budapest.

Exemple 2

Cet exemple utilise le système de complémentation et les vecteurs pIX128 et pAD121 décrits dans l'exemple 1.

1. Choix d'une séquence peptidique

La séquence du peptide insérée dans la piline est :
Asn-Asn-Pro-His-Arg-Ile-Leu
Il s'agit d'une séquence de sept acides aminés, qui appartient à l'hémagglutinine du virus de l'Influenza. Elle est localisée dans l'antigène B de l'hémagglutinine 1 du virus. Cette séquence est dénommée ci-après Flu2.

2. Clonage

24

Un DNA synthétique codant pour ce peptide a été synthétisé, de sorte qu'il permette un clonage directionnel et en phase dans les sites KpnI et XbaI des vecteurs pIX128 et pAD121. Le troisième acide aminé de l'épitope est une proline, et la séquence de restriction KpnI GGTACC introduit un acide aminé proline, codé par le triplet CCx. Afin d'éviter la proximité de deux prolines, le nucléotide T en 5′ du DNA synthétique est introduit. Le codon deviendra CTG, et introduira l'acide aminé leucine. Le palindrome reconnu par KpnI est supprimé. Le choix de la séquence nucléotidique tient compte du choix préférentiel des codons par E. Coli. Cette séquence est :

```
              Leu Asn Asn Pro His Arg Ile Leu
        5'      TG AAC AAC CCG CAC AGA ATT
        3' CAT GAC TTG TTG CGC GTG TCT TAA GAT C
           ------                      ------
           KpnI                        XbaI
```

Ce DNA fut cloné dans les sites KpnI et XbaI du pIX128, générant le pIX142 d'une part, ainsi que dans ces mêmes sites du pAD121, générant le plasmide pAD126 d'autre part. La procédure de clonage est classique : double restriction du plasmide par KpnI et XbaI, suivie de sa purification sur gel d'agarose, et ligation en présence d'un large excès de DNA synthétique hybridé. Le criblage des recombinants est facilité par la perte du site KpnI. Différents candidats ont été isolés et vérifiés par séquençage.

### 3. Production de pili

Chacun des plasmides recombinants est introduit par transformation dans une bactérie E. coli qui contient le plasmide pIX211, EC294 (pIX211). Pour rappel, ce plasmide porte les gènes dits "helpers" codant pour l'excrétion et l'assemblage du pili K88.

La piline codée par le pIX142 est longue de 264aa (acides aminés), soit la taille exacte de la piline K88ab et ad. Pour sa part, la piline déterminée par le pAD126 est longue de 262 aa, soit la taille de la piline native de K88ac. D'une certaine manière, l'expression de ces gènes va révéler l'importance de la taille globale de la piline sur son assemblage en pili.

### a. Test sur colonie

Les figures 16 et 17 montrent clairement que les bactéries productrices des pilines IX142 et AD126 possèdent une grande quantité d'antigènes K88 à leur surface. En effet, les colonies qui contiennent le pIX142 (ligne 5, clones 4, 5 et 6 et ligne 6, clones 1, 2 et 3) et celles qui hébergent le pAD126 (ligne 2, clones 4, 5 et 6) sont fortement reconnues par le sérum polyclonal, aussi bien que par l'anticorps monoclonal. L'épitope reconnu par cet anticorps monoclonal est structural. Sa reconnaissance des pilines pIX142 et pAD126 indique donc qu'elles sont associées en pili.

### b. Elisa

Des rasats de cultures productrices des pilines IX142 et AD122 ont été analysés par Elisa, et détectés soit par des monoclonaux, soit par des polyclonaux. On peut estimer que la concentration en antigènes pili des extraits du pIX142 est la même que celle des extraits des cultures de référence productrices du pili sauvage : une moyenne de 18 micro g/ml d'antigènes IX142 sont détectés dans trois rasats thermiques indépendants, pour 19 micro g/ml d'antigènes IX120 (cf. Tableau 4). Une proportion comparable est observée dans les rasats de la culture productrice du pili AD126.

Des rasats mécaniques d'une culture productrice des antigènes IX142 (60 litres de culture en fermenteur) permet de purifier 4 mg d'antigènes. Le gel PAGE de cette préparation montre que la bande protéique majeure est celle de la piline.

### c. Gels et blots

A partir d'un extrait de pili, les pilines IX142 et AD126 sont aisément identifiées sur un gel PAGE et sur un western blot qui utilise un sérum anti-pili comme moyen de détection. Leur migration est sensiblement identique à celle des sous-unités non modifiées IX120 ou AD120. La concentration en antigène déterminée par un Elisa est compatible avec l'intensité de la bande formée par la piline sur le gel.

4. Antigénicité du peptide fusionné au pilus

Un peptide qui reproduit la séquence N-N-P-H-R-I-L du Flu2 a été synthétisé et des anticorps monoclonaux dirigés spécifiquement contre ce peptide synthétique ont été sélectionnés. Par Elisa, on a montré qu'un de ces monoclonaux réagit plus contre l'antigène IX142 que contre le contrôle IX120.

Exemple 3

1. Choix du peptide

La séquence fusionnée aux pili dans cet exemple est la séquence de l'hormone somatostatine, aussi appelée facteur inhibant la libération de l'hormone de croissance. La composition de ce peptide long de 14 acides aminés, la composition du DNA synthétique qui en détermine la synthèse et leurs caractéristiques sont :

```
          1       3       5                    10             14
       Ala-Gly-Cys-Lyx-Asn-Phe-Phe-TrP-Lys-Thr-Phe-Thr-Ser-Cys
    5'     CT GCA GGC TGT AAG AAC TTT TTC TGG AAG ACG TTC ACC AGC TGT
    3' CAT GA CGT CCG ACA TTC TTG AAA AAG ACC TTC TGC AAG TGG TCG ACA GAT CT
       ----=--------       ----......---   ----......---   ---------========
       KpnI  PstI          XmnI            XmnI            PvuII  XbaI
```

Le peptide contient deux cystéines en position 3 et 14 respectivement, qui peuvent former un pont disulfure, et une forte proportion de résidus aromatiques (phénylalanine et tryptophane) et chargés (lysine).

2. Clonage

Le DNA synthétique porte à ses extrémités 5′ et 3′ des séquences protubérantes complémentaires aux extrémités cohésives des sites KpnI et XbaI respectivement. Le choix des codons, tout en respectant le choix préférentiel de E. coli, permet d'introduire des sites de restriction pour PstI (CTGCAG), XmnI (GAA----TTC) et PvuI (CAGCTG). Ces sites sont utiles pour le criblage des plasmides recombinants ayant intégré cet insert, et offrent diverses possibilités de manipulations de la séquence de la somatostatine.

Après son clonage dans un vecteur pUC18 et sa vérification par un séquençage, le DNA de synthèse de la somatostatine a été cloné dans les vecteurs pIX128 (S1) et pAD121 (S2). Les plasmides recombinants sont notés pIXSM pour le clonage en S1 et pADSM pour l'insertion en S2. Outre la vérification des modèles de restriction, notamment pour les sites KpnI, XbaI, XmnI, PstI et PvuII, la séquence nucléotidique de leur insert et des régions qui le flanquent a été confirmée.

3. Production de pili

Le pIXSM, ainsi que le pADSM, furent introduits dans la souche EC294 (pIX211) pour tester l'assemblage d es pilines recombinantes en pili. La piline codée par le pIXSM a une taille de 272 acides aminés, tandis que celle codée par le pADSM est longue de 270 acides aminés.

a. Test sur colonie

Un test immunologique sur bactérie est présenté à la figure 16. Les colonies bactériennes qui contiennent le pIXSM (ligne 6, clones 4, 5 et 6) sont reconnues par le sérum polyclonal anti-K88. Cette réaction est habituellement moins intense que celle observée sur le contrôle positif, résultat qui suggère la présence de moins d'antigènes K88 à la surface de ces bactéries. Les colonies porteuses du pADSM sont très nettement reconnues par ce sérum, suggérant une grande quantité de pili accrochés à la bactérie (ligne 3, clones 1, 2 et 3).

Le même test réalisé cette fois avec des monoclonaux anti-K88, qui reconnaissent la piline associée en pili, est présenté à la figure 17. Une réponse moindre est observée contre la bactérie productrice de piline IXSM. La réaction de l'anticorps monoclonal contre les antigènes ADSM est intense; cela prouve que les pilines recombinantes, contenant la séquence somatostatine, sont effectivement assemblées en pili.

b. ELISA

Un Elisa dont le but est de doser la quantité d'antigène K88 dans les rasats thermiques des souches qui expriment les pilines IXSM et ADSM (pilines qui sont codées respectivement par le plasmide pIXSM et le plasmide pADSM) permet de conclure que les extraits contiennent moins de 0,2 micro g d'antigènes par ml, soit une chute à 1 % de ce qui est habituellemnt extrait des souches non modifiées (cf. Tableau 4).

Ces résultats paraissent contradictoires avec les observations des tests immunologiques sur colonies. La faible quantité d'antigènes K88 dans les extraits pourrait cependant s'expliquer si les conditions d'extraction appliquées aux pili recombinants détruisaient leur structure quaternaire.

4. Antigénicité du peptide

Un anticorps monoclonal spécifique à la somatostatine a donc été obtenu. Un Elisa sur des rasats de souches productrices de piline AD121 et de piline ADSM adsorbé sur une plaque, montre clairement une réaction sur le rasat ADSM, et une très faible réponse sur les contrôles négatifs. Ceci prouve que cet antigène est bien présent dans les rasats de cette souche.

Tableau 4

| Nom du plasmide | Concentration protéines micro g/ml | Concentration Ag K88 micro g/ml | % |
|---|---|---|---|
| pIX120 | 100 | 19 | 100 |
| pIX142 | 128 | 18 | 95 |
| pIXSM | 130 | < 0,2 | < 1 |
| pAD121 | 202 | 23 | 100 |
| pAD126 | 171 | 18 | 78 |
| pADSM | 164 | < 0,2 | < 1 |

Tableau 4 : Concentrations en antigènes K88 (en micro g/ml) et en protéines totales (en micro g/ml; méthode de lowry) dans différents rasats thermiques déterminées par Elisa. La quantité d'antigènes K88 produite par les souches recombinantes est estimée par rapport à la quantité purifiée des souches sauvages.

Exemple 4

Le présent exemple est destiné à montrer que des pilines recombinantes dont les deux régions variables S1 et S2 contiennent une séquence étrangère aux pili, peuvent être construites et exprimées. Ceci est réalisé en combinant sur une même piline les séquences S1 des dérivés K88ac, avec les séquences S2 des dérivés K88ad.

1. Choix des épitopes

Les gènes des pilines recombinantes contiennent la séquence S1-Flu2 du plasmide pIX142, et soit S2-Flu1 du pAD122 (essai 1) soit S2-Flu2 du pAD126 (essai 2). Les pilines codées par ces gènes sont formées d'une partie de la piline IX142, depuis leur extrémité amine jusqu'à et y compris la séquence S1, suivie de la séquence C-terminale de la piline AD122 (essai 1) ou AD126 (essai 2).

27

2. Clonage

Comme tous les dérivés du pIX120 et du pAD120, les plasmides pIX142, pAD122 et pAD126 contiennent deux sites de restriction BgII. L'un est localisé dans le gène de résistance à l'ampicilline, et le second est situé dans le gène de la piline, précisément entre les régions S1 et S2 (référence est faite aux figures 3, 4, 5 et 6). Pour construire ces gènes recombinants, deux types de fragments sont purifiés :

- du pIX142 : le fragment BgII qui porte la fin du gène AmpR, l'origine de réplication et le début du gène de la piline, y compris S1-Flu2;
- du pAD122 (essai 1) ou pAD126 (essai 2) : le fragment BgII qui contient la fin du gène piline (y compris S2-Flu1 ou S2-Flu2) et le début du gène AmpR.

La ligation de ces deux types de fragment restaure les gènes de résistance à l'ampicilline et de la piline. Ces plasmides sont respectivement pIX143, dérivé du pAD122 (essai 1), et pIX144, formé à partir du pAD126 (essai 2).

3. Production de pili

La taille de la piline IX143 est de 266 acides aminés, et 262 acides aminés pour IX144.

La production de bactéries porteuses de pili est obtenue après introduction de chacun des plasmides pIX143 et pIX144 dans la souche d'expression EC294 (pIX211).

Conclusions générales des exemples

L'ensemble des résultats expérimentaux montrent que :

1. Les régions variables S1 et S2 peuvent être délétées de la piline, sans compromettre la synthèse de pili.

2. Sur cette base, deux gènes pilines "vecteurs" dans lesquels l'insertion de DNA étranger est facilitée par la présence de sites de restriction uniques, ont été construits.

3. Des DNA codant pour des séquences peptidiques différentes par leur longeur et leur composition peuvent être introduites, par génie génétique, dans ces vecteurs, et déterminer des pilines recombinantes. Par complémentation intergénique, ces pilines peuvent être assemblées en pili attachés à la surface de la bactérie. Ceci est vrai pour deux régions différentes de la piline. La tolérance observée dans les exemples précédents pour chacune des régions est d'au moins de -4 à +10 pour S1 et de -6 à +6 pour S2.

4. Les pili recombinants peuvent être purifiés selon les mêmes méthodes que celles employées pour les pili sauvages.

5. Le peptide fusionné au pili est reconnu par des anticorps qui lui sont spécifiques et son antigénicité est amplifiée.

6. Le pili recombinant utilisé comme antigène induit deux types d'anticorps : des anticorps anti-pili, mais aussi des anticorps dirigés contre le peptide fusionné au pili. Ce peptide est rendu immunogénique. Des pili recombinants peuvent donc être utilisés dans des vaccins sous-unitaires.

Tableau 1 : Liste des plasmides

| | Nom | Description |
|---|---|---|
| Vecteurs : | pBR322 | $Ap^R Tc^R$ (1) |
| | pBR322E | = pBR322 dépourvu du site EcoR1 |
| | pACYC184 | $Cm^R Tc^R$ (2) |
| | pUR222 | $Ap^R$ Lac alpha (3) |
| | pUC8 | $Ap^R$ Lac alpha |
| | pUC18 | $Ap^R$ Lac alpha |
| | pUCtgl30 | polylinker du M13 tgl30 remplace le polylinker du pUC18 |
| K88ac : | pIX102 | pBR322 ::K88ac clonage d'un fragment HindIII de 12 kb du plasmide pK88ac |
| | pIX105 | restriction EcoR1 partielle du pIX102. Insert de 7kb |
| | pIX115 | idem pIX105 mais l'insert K88 est flanqué de deux sites HindIII |
| | pIX120 | Fragment DraI de K88, contenant le gène de la sous-unité, cloné dans le site HindIII/klenow du pBR322; délétion BamH1-Aval |
| | pIX126 | =pIX120 dont le fragment SacI/BssHII est remplacé par le linker du pUCtgl30 |
| | pIX128 | =pIX120 dont le fragment SacI/BssHII est remplacé par un adaptateur synthétique |
| | pIX130 | pIX126 contenant un épitope de l'hémagglutinine du virus de l'Influenza (Flul) dans le linker tgl30. |
| | pIX131 | =pIX130 avec la partie SacI-KpnI du linker du pUC18 |

| Nom | Description |
|---|---|
| pIX134 | pIX120 dont le fragment Bgll-Bgll porteur de l'extrémité 3' du gène de la piline ac est remplacé par le fragment Bgll-Bgll équivalent du pAD120. |
| pIX135 | pAD120 dont le fragment Bgll-Bgll porteur de l'extrémité 3' du gène de la piline ad est remplacé par le fragment Bgll-Bgll équivalent du pIX120. |
| pIX136 | =pIX128 contenant un épitope de l'hémagglutinine du virus de l'Influenza (Flu1). |
| pIX139 | =pIX136 avec la partie SacI-kpnI du pUC18. |
| pIX211 | =pACYC184 contenant l'opéron K88ac, délété du gène de la sous-unité, cloné dans le site HindIII |
| pIX142 | =pIX128 avec le DNA synthétique codant pour un épitope de l'hémagglutinine du virus de l'Influenza (Flu2) cloné dans les sites KpnI et XbaI |
| pIXSM | =pIX128 avec le DNA synthétique codant pour la somatostatine cloné dans les sites KpnI et XbaI |
| K88ad : pAD102 | pBR322::K88ad - clonage de l'opéron K88ad |
| pAD120 | id pIX120, mais la sous-unité appartient au sérotype K88ad |
| pUC81 | Délétion HindII/HindIII du pUC8 |
| pUC82 | =pUC81 contenant le fragment ECOR1-XhO2 du gène piline de pAD120 |

30

| Nom | Description |
|---|---|
| pUC83 | = pUC82 dont le fragment Hind II-BamH1 a été remplacé par un adaptateur synthétique |
| pUC84 | le fragment XhO2 contenant la partie 3' du gène de la sous-unité K88ad du pADd120 est cloné dans le site BamH1 du pUC83. |
| pAD121 | =pAD120 dont le fragment Hind II-BamH1 a été remplacé par un adaptateur synthétique |
| pAD122 | =pAD121 contenant l'ADN codant pour l'épitope de l'Influenza (Flu1) |
| pAD126 | =pAD121 avec le DNA synthétique codant pour un épitope de l'hémag-glutinine du virus de l'Influenza (Flu2), cloné dans les sites KpnI et XbaI |
| pADSM | =pAD121 avec le DNA synthétique codant pour la somatostatine cloné dans les sites KpnI et XbaI |

Les références sont :

(1) Bolivar et al, 1977, Gene ,2 95-113

(2) Chang A.C.Y. et Cohen S.N., 1978, J. Bacteriol. 134, 1141-1156

(3) Rütter et al, 1981, Nucl. Acids Res., 9, 4087-4098

Légende des figures

figure 1

Analyse des sous-unités pilines produites par diverses souches E. coli, en gel de polyacrylamide -SDS et en Western blot.

figure 1a

Piste R : références de PM : phosphorylase b-94KD, albumine 67KD, ovalbumine 43KD, anhydrase carbonique 30kD, inhibiteur de la trypsine 20 kD, alpha-lactalbumine 14.4.kD

1. rasat purifié de K88ac
2. rasat brut de K88ab NL
3. rasat brut de K88ab US
4. rasat brut de K88ac

EP 0 264 150 B1

5. rasat brut de EC294 (pIX115)
6. rasat brut de EC294 (pIX120)(pIX211)
7. rasat brut de K88ad NL
8. rasat brut de K88ad US
9. rasat brut de EC294 (pAD102)
10. rasat brut de EC294 (pAD120)(pIX211)

figure 1b

Western blot du gel de la figure 1a.

figure 2

Carte de restriction de l'opéron K88ac cloné dans le plasmide pBR322. La position des sites de restriction pour quelques enzymes courants est donnée, ainsi que la carte génétique de l'opéron. La taille des 6 protéines est donnée en kD. La protéine de 17.6 kD est putative.

figure 3

Séquence du gène de la sous-unité K88ac cloné dans le vecteur pBR322 et sa traduction en peptide, ainsi que la position de quelques sites de restriction remarquables.
    La séquence du vecteur est écrite en lettres minuscules.
RBS : ribosome binding site.
$P_1$ : promoteur cryptique du vecteur.

figure 4

Carte de restriction du pIX120. La position du gène de la sous-unité K88ac (K88), du gène de résistance à l'ampicilline (AP) et de l'origine de réplication du pBR322 (Ori) est donnée.

figure 5

Séquence du gène de la sous-unité K88ad US et sa traduction en peptide ainsi que la position de quelques sites de restriction remarquables.

figure 6

Carte de restriction du pAD120 avec le gène de la sous-unité K88AD, de résistance à l'ampicilline (AMP) et l'origine de réplication du plasmide (Ori).

figure 7

Schéma de la complémentation intergénique pour K88ac : les gènes de l'opéron K88 (2), porté par le pIX115, ont été sous clonés sur deux vecteurs : pIX211, dérivé du pACYC184, porte les génes des protéines d'ancrage et d'assemblage des pili et pIX120, dérivé du pBR322, contenant le seul gène de la sous unité (1). $P_1$ est un promoteur cryptique du vecteur. ORI : origine de réplication autonome.

figure 8

Profil d'hydrophilicité de la piline K88ac, d'après l'algorithme de Doolittle. Les zones particulièrement hydrophiles sont hachurées.

figure 9

Comparaison des séquences protéiques des trois sérotypes K88 et de leurs variants. Seules les différences en acides aminés sont notées. Des délétions symbolisées par un "-" sont introduites pour faire correspondre au mieux les séquences peptidiques.

32

figure 10

Comparaison des spectres d'hydrophilicité des différents sérotypes et de leurs variants suivant l'algorithme de Doolittle.

figure 11

Manipulations de la région S1. Les plasmides pIX126 (2), pIX130 (3), pIX131 (4), pIX128 (5), pIX136 (6) et pIX139 (7) sont dérivés du gène K88ac du pIX120 (1). Les acides aminés variables de $S_1$ sont identifiés par un astérisque *.

figure 12

Test immunologique sur colonies des souches recombinantes.

```
    rangée  1 - EC294 (pIX211)(pIX120) souche productrice de pili K88ac,
               utilisée comme témoin positif.
    rangée  2 - EC294 (pIX120)(pACYC184) et EC294 (pUR222)(pIX211) deux
               témoins négatifs.
    rangée  3 - EC294 (pIX211)(pIX126)
    rangée  4 - EC294 (pIX211)(pIX130)
    rangée  5 - EC294 (pIX211)(pIX131)
```

```
            rangée  6 - EC294 (pIX211)(pIX128)
            rangée  7 - EC294 (pIX211)(pIX136)
            rangée  8 - EC294 (pIX211)(pIX139)
            rangée  9 - EC294 (pIX211)(pAD120)
            rangée 10 - EC294 (pIX211)(pAD121)
            rangée 11 - EC294 (pIX211)(pAD122)
```

L'anticorps est un polyclonal anti K88ac. Cet anticorps est reconnu par un anti-lapin couplé à la peroxydase.

figure 13 - Etapes de clonage permettant le remplacement de la région S2 par un adaptateur synthétique. Les cartes de restriction des différents intermédiaires qui ont permis la construction du pAD121 sont présentées à la figure 13a : pUC8, figure 13b : pUC81, figure 13c : pUC82, figure 13d : pUC83, figure 13e : pUC84 et enfin figure 13 f : pAD121.

figure 14 - Manipulation de la région S2 : les plasmides pAD121 (2), et pAD122 (3) sont dérivés du plasmide pAD120 (1).

Figure 15a - gel de PAGE-SDS d'un rasat brut des souches

    1 - EC294 (pAD120)(pIX211)
    2 - EC294 (pAD121)(pIX211)
    3 - EC294 (pAD122)(pIX211)

figure 15b

Western blot des rasats bruts séparés sur le gel de la figure 15a.

figure 16 - Test immunologique sur diverses bactéries recombinantes avec un sérum anti-K88. Trois colonies isolées de chaque type de bactéries ont été transférées sur le filtre. A l'exception des clones de la ligne 7 qui contiennent le pACYC184 au lieu du pIX211, ce sont des souches EC294 (pIX211) porteuse de l'un des plasmides recombinants suivants :

```
        clones : 1, 2 et 3          4, 5 et 6
   Ligne 1 :      pAD120            pAD121
   Ligne 2 :      pAD122            pAD126
   Ligne 3 :      pADSM             pUR222
   Ligne 4 :      pIX120            pIX128
   Ligne 5 :      pIX136            pIX142



   Ligne 6 :      pIX142            pIXSM
   Ligne 7 :      (pIX120)(pACYC184)
```

Figure 17 - La légende est la même que pour la figure 16, sauf que le moyen de détection est un anticorps monoclonal anti-K88.

Figure 18 - Elisa des rasages thermiques des souches contenant le plasmide pIX120 (1), pIX128 (2) et pIX136 (3), détecté par un anticorps monoclonal anti-K88. La référence (R) est à 1 micro g/ml. En abscisse se trouve le taux de dilution, en ordonnée la densité optique.

Figure 19 - Réaction sur Elisa de deux monoclonaux anti-Flu1 contre des pili IX128 (I) et IX136 (II). En abscisse se trouve le taux de dilution, en ordonnée la densité optique.

Figure 20 - Réponse humorale des quatre rats immunisés par des concentrations croissantes d'antigènes AD122, contre le pili K88. La quantité d'antigènes par dose est reportée en abscisse. L'absorbance à la dilution 10 de chaque sérum contre le pili est en ordonnée. En abscisse se trouve la quantité de micro g de pili injectés.

Figure 21 - Même légende que la figure 20, sinon que l'antigène adsorbé est le peptide synthétique Flu1. En abscisse se trouve la quantité de micro g de pili injectés et l'absorbance à la dilution 10 de chaque sérum contre le pili est ordonnée.

**Revendications**

**Revendications pour les Etats contractants suivants : AT, BE, CH, DE, FR, GB, IT, LI, LU, NL, SE**

1. Microorganismes recombinants ayant une membrane externe qui porte des pili dont la composition est modifiée par au moins deux changements de la séquence protéique de la sous-unité ou piline, changements qui résultent de l'insertion dans le gène codant pour la sous-unité d'au moins deux fragments d'ADN codant pour un peptide ou une protéine hétérologue, faisant partie d'une région antigénique du virus de l'Influenza et/ou de l'hémagglutinine du virus de l'Influenza et/ou étant un peptide de l'hormone somatostatine.

2. Pili isolés de microorganismes recombinants ayant une membrane externe qui porte des pili caractérisés en ce que la composition de ces pili est modifiée par au moins deux changements de la séquence protéique de la sous-unité ou piline, changements qui résultent de l'insertion dans le gène codant pour la sous-unité d'au moins deux fragments d'ADN codant pour un peptide ou une protéine hétérologue, faisant partie d'une région antigénique du virus de l'Influenza et/ou de l'hémagglutinine du virus de l'Influenza et/ou étant un peptide de l'hormone somatostatine.

3. Procédé pour obtenir des pili selon la revendication 2 caractérisé en ce que ces changements résultent de l'introduction dans le gène codant pour la sous-unité d'un pilus, d'au moins deux fragments d'ADN codant pour un peptide ou une protéine hétérologue, faisant partie d'une région antigénique du virus de l'Influenza et/ou de l'hémagglutinine du virus de l'Influenza et/ou étant un peptide de l'hormone somatostatine.

4. Procédé selon la revendication 3 caractérisé en ce que le fragment d'ADN codant pour un peptide ou une protéine hétérologue, faisant partie d'une région antigénique du virus de l'Influenza et/ou de l'hémagglutinine du virus de l'Influenza et/ou étant un peptide de l'hormone somatostatine, est un fragment d'ADN synthétique.

5. Procédé selon l'une des revendications 3 ou 4 caractérisé en ce que l'ADN codant est inséré dans une zone du gène codant pour la sous-unité d'un pilus qui correspond à une zone exposée au milieu environnant de la séquence protéinique des pilines formant le pilus.

6. Procédé pour obtenir des microorganismes ayant une membrane externe qui porte des pili tels qu'obtenus selon l'une quelconque des revendications 3, 4 ou 5.

7. Utilisation de protéines obtenues par au moins deux modifications de la séquence protéique de la sous-unité de pili ou piline, selon la revendication 2, en tant que produits pour la santé animale ou pour la santé humaine.

8. Utilisation de pili, de pilines selon la revendication 2 ou de microorganismes selon la revendication 1, pour l'obtention d'un produit pharmaceutique destiné au traitement médical et/ou prophylactique en santé humaine ou animale.

9. Utilisation de pili, de pilines selon la revendication 2 ou de microorganismes selon la revendication 1, pour la fabrication de moyen destiné à la préparation de tests immunologiques.

**Revendications pour les Etats contractants suivants : ES, GR**

1. Procédé pour obtenir des pili, isolés de microorganismes recombinants, dont la composition a été modifiée par au moins deux changements de la séquence protéique de la sous-unité ou piline caractérisé en ce que ces changements résultent de l'introduction dans le gène codant pour la sous-unité d'un pilus, d'au moins deux fragments d'ADN codant pour un peptide ou une protéine hétérologue, faisant partie d'une région antigénique du virus de l'Influenza et/ou de l'hémagglutinine du virus de l'Influenza et/ou étant un peptide de l'hormone somatostatine.

2. Procédé selon la revendication 1 caractérisé en ce que le fragment d'ADN codant pour un peptide ou une protéine hétérologue, faisant partie d'une région antigénique du virus de l'Influenza et/ou de l'hémagglutinine du virus de l'Influenza et/ou étant un peptide de l'hormone somatostatine, est un fragment d'ADN synthétique.

3. Procédé selon les revendications 1 ou 2 caractérisé en ce que l'ADN codant est inséré dans une zone du gène codant pour la sous-unité d'un pi'lus qui correspond à une zone exposée au milieu environnant de la séquence protéinique des pilines formant le pilus.

4. Procédé pour obtenir des microorganismes recombinants ayant une membrane externe qui porte des pili tels qu'obtenus selon l'une quelconque des revendications 1, 2 ou 3, caractérisé en ce que la composition des pili a été modifiée par au moins deux changements de la séquence protéique de la sous-unité ou piline, ces changements résultant de l'introduction dans le gène codant pour la sous-unité d'un pilus, d'au moins deux fragments d'ADN codant pour un peptide ou une protéine hétérologue, faisant partie d'une région antigénique du virus de l'Influenza et/ou de l'hémagglutinine du virus de l'Influenza et/ou étant un peptide de l'hormone somatostatine.

5. Utilisation de protéines obtenues par au moins deux modifications de la séquence protéique de la sous-unité de pili ou piline, obtenus selon l'une quelconque des revendications 1, 2 ou 3, en tant que produits pour la santé animale ou pour la santé humaine.

6. Utilisation de pili, de pilines obtenus selon l'une quelconque des revendications 1, 2 ou 3 ou de microorganismes obtenus selon la revendication 4, pour l'obtention d'un produit pharmaceutique destiné au traitement médical et/ou prophylactique en santé humaine ou animale.

7. Utilisation de pili, de pilines obtenus selon l'une quelconque des revendications 1, 2 ou 3 ou de microorganismes obtenus selon la revendication 4, pour la fabrication de moyen destiné à la préparation de tests immunologiques.

**Claims**
**Claims for the following Contracting States : AT, BE, CH, DE, FR, GB, IT, LI, LU, NL, SE**

35

EP 0 264 150 B1

1. Recombinant microorganisms having an outer membrane which bears pili, the composition of which is modified by at least two changes in the protein sequence of the subunit or pilin, these changes resulting from the insertion, into the gene coding for the subunit, of at least two DNA fragments coding for a heterologous peptide or a heterologous protein forming part of an antigenic region of the influenza virus and/or of the haemagglutinin of the influenza virus and/or being a peptide of the hormone somatostatin.

2. Pili isolated from recombinant microorganisms having an outer membrane which bears pili, characterised in that the composition of these pili is modified by at least two changes in the protein sequence of the subunit or pilin, these changes resulting from the insertion, into the gene coding for the subunit, of at least two DNA fragments coding for a heterologous peptide or a heterologous protein forming part of an antigenic region of the influenza virus and/or of the haemagglutinin of the influenza virus and/or being a peptide of the hormone somatostatin.

3. Method for obtaining pili according to Claim 2, characterised in that these changes result from the introduction, into the gene coding for the subunit of a pilus, of at least two DNA fragments coding for a heterologous peptide or a heterologous protein forming part of an antigenic region of the influenza virus and/or of the haemagglutinin of the influenza virus and/or being a peptide of the hormone somatostatin.

4. Method according to Claim 3, characterised in that the DNA fragment coding for a heterologous peptide or a heterologous protein forming part of an antigenic region of the influenza virus and/or of the haemagglutinin of the influenza virus and/or being a peptide of the hormone somatostatin is a synthetic DNA fragment.

5. Method according to one of Claims 3 and 4, characterised in that the coding DNA is inserted into a region of the gene coding for the subunit of a pilus which corresponds to a region exposed to the surrounding medium of the protein sequence of the pilins forming the pilus.

6. Method for obtaining microorganisms having an outer membrane which bears pili as obtained according to any one of Claims 3, 4 and 5.

7. Use of proteins obtained by at least two modifications of the protein sequence of the subunit of pili or pilin, according to Claim 2, as products for the animal health or human health field.

8. Use of pili or pilins according to Claim 2 or of microorganisms according to Claim 1, for obtaining a pharmaceutical product intended for medical and/or prophylactic treatment in the field of human or animal health.

9. Use of pili or pilins according to Claim 2, or of microorganisms according to Claim 1, for the manufacture of means intended for the preparation of immunological tests.

**Claims for the following Contracting States : ES, GR**

1. Method for obtaining pili, isolated from recombinant microorganisms, the composition of which has been modified by at least two changes in the protein sequence of the subunit or pilin, characterised in that these changes result from the introduction, into the gene coding for the subunit of a pilus, of at least two DNA fragments coding for a heterologous peptide or a heterologous protein forming part of an antigenic region of the influenza virus and/or of the haemagglutinin of the influenza virus and/or being a peptide of the hormone somatostatin.

2. Method according to Claim 1, characterised in that the DNA fragment coding for a heterologous peptide or a heterologous protein forming part of an antigenic region of the influenza virus and/or of the haemagglutinin of the influenza virus and/or being a peptide of the hormone somatostatin is a synthetic DNA fragment.

3. Method according to Claims 1 or 2, characterised in that the coding DNA is inserted into a region of the gene coding for the subunit of a pilus which corresponds to a region exposed to the surrounding medium of the protein sequence of the pilins forming the pilus.

36

4. Method for obtaining recombinant microorganisms having an outer membrane which bears pili as obtained according to any one of Claims 1, 2 or 3, characterised in that the composition of the pili has been modified by at least two changes in the protein sequence of the subunit or pilin, these changes resulting from the introduction into the gene coding for the subunit of a pilus, of a least two DNA fragments coding for a heterologous peptide or a heterologous protein forming part of an antigenic region of the influenza virus and/or of the haemagglutinin of the influenza virus and/or being a peptide of the hormone somatostatin.

5. Use of proteins obtained by at least two modifications of the protein sequence of the subunit of pili or pilin, obtained according to any one of Claims 1, 2 and 3, as products for the animal health or human health field.

6. Use of pili or pilins obtained according to any one of Claims 1, 2 and 3 or microorganisms obtained according to Claim 4, for obtaining a pharmaceutical product intended for medical and/or prophylactic treatment in the field of human or animal health.

7. Use of pili or pilins obtained according to any one of Claims 1, 2 and 3 or microorganisms obtained according to Claim 4, for the manufacture of means intended for the preparation of immunological tests.

**Patentansprüche**
**Patentansprüche für folgende Vertragsstaaten : AT, BE, CH, DE, FR, GB, IT, LI, LU, NL, SE**

1. Rekombinante Mikroorganismen mit einer äußeren Membran, die Pili trägt, deren Zusammensetzung durch wenigstens zwei Veränderungen der Proteinsequenz der Untereinheit oder Pilin modifiziert ist, wobei die Veränderungen, die aus dem Einfügen in das für die Untereinheit kodierende Gen von wenigstens zwei für ein heterologes Peptid oder ein heterologes Protein kodierenden DNS-Fragmenten hervorgehen, einen antigenen Bereich des Influenzavirus und/oder des Hämagglutinins des Influenzavirus bilden und/oder ein Peptid des Somatostatinhormons sind.

2. Isolierte Pili von rekombinanten Mikroorganismen mit einer äußeren Membran, die Pili trägt, dadurch gekennzeichnet, daß die Zusammensetzung dieser Pili durch wenigstens zwei Veränderungen der Proteinsequenz der Untereinheit oder Pilin modifiziert ist, wobei die Veränderungen, die aus dem Einfügen in das für die Untereinheit kodierende Gen von wenigstens zwei für ein heterologes Peptid oder ein heterologes Protein kodierenden DNS-Fragmenten hervorgehen, einen antigenen Bereich des Influenzavirus und/oder des Hämagglutinins des Influenzavirus bilden und/oder ein Peptid des Somatostatinhormons sind.

3. Verfahren zum Erhalt der Pili gemäß Anspruch 2, dadurch gekennzeichnet, daß diese Veränderungen aus dem Einfügen in das für die Untereinheit eines Pilus kodierenden Gens von wenigstens zwei für ein heterologes Peptid oder ein heterologes Protein kodierende DNS-Fragmenten hervorgehen, die einen antigenen Bereich des Influenzavirus und/oder des Hämagglutinins des Influenzavirus bilden und/oder ein Peptid des Somatostatinhormons sind.

4. Verfahren nach Anspruch 3, dadurch gekennzeichnet, daß das für ein heterologes Peptid oder ein heterologes Protein kodierende DNS-Fragment, das einen antigenen Bereich des Influenzavirus und/oder des Hämagglutinins des Influenzavirus bildet und/oder ein Peptid des Somatostatinhormons ist, ein synthetisches DNS-Fragment ist.

5. Verfahren nach einem der Ansprüche 3 oder 4, dadurch gekennzeichnet, daß die kodierende DNS eingefügt wird in einen Bereich des für die Untereinheit eines Pilus kodierenden Gens, der einem dem Umgebungsmedium ausgesetzten Bereich der Proteinsequenz der Piline, die den Pilus bilden, entspricht.

6. Verfahren zum Erhalt von Mikroorganismen mit einer äußeren Membran, die die nach einem der Ansprüche 3, 4 oder 5 erhaltenen Pili tragen.

7. Verwendung der durch wenigstens zwei Modifikationen der Proteinsequenz der Untereinheit der Pili oder Pilin erhaltenen Proteine nach Anspruch 2 als Produkte für die tierische oder menschliche

37

Gesundheit.

**8.** Verwendung von Pili, von Pilinen nach Anspruch 2 oder von Mikroorganismen nach Anspruch 1 zum Erhalt eines pharmazeutischen Produkts, das für die medizinische und/oder prophylaktische Behandlung der menschlichen oder tierischen Gesundheit bestimmt ist.

**9.** Verwendung von Pili, von Pilinen nach Anspruch 2 oder von Mikroorganismen nach Anspruch 1, für die Herstellung eines für die Durchführung von immunologischen Tests bestimmten Mittels.

**Patentansprüche für folgende Vertragsstaaten : ES, GR**

**1.** Verfahren zum Erhalt von Pili, die von rekombinanten Mikroorganismen isoliert wurden, deren Zusammensetzung durch wenigstens zwei Veränderungen der Proteinsequenz der Untereinheit oder Pilin modifiziert wurde, dadurch gekennzeichnet, daß diese Veränderungen aus dem Einfügen in das für die Untereinheit eines Pilus kodierenden Gens von wenigstens zwei für ein heterologes Peptid oder ein heterologes Protein kodierenden DMS-Fragmenten hervorgehen, die einen antigenen Bereich des Influenzavirus und/oder des Hämagglutinins des Influenzavirus bilden und/oder ein Peptid des Somatostatinhormons sind.

**2.** Verfahren nach Anspruch 1, dadurch gekennzeichnet, daß das für ein heterologes Peptid oder ein heterologes Protein kodierende DMS-Fragment, das einem antigenen Bereich des Influenzavirus und/oder des Hämagglutinins des Influenzavirus bildet und/oder ein Peptid des Somatostatinhormons ist, ein synthetisches DMS-Fragment ist.

**3.** Verfahren nach den Ansprüchen 1 oder 2, dadurch gekennzeichnet, daß die kodierende DMS eingefügt wird in einen Bereich des für die Untereinheit eines Pilus kodierenden Gens, der einem dem Umgebungsmedium ausgesetzten Bereich der Proteinsequenz der den Pilus bildenen Piline entspricht.

**4.** Verfahren zum Erhalt von rekombinanten Mikroorganismen mit einer äußeren Membran, die Pili, wie nach einem der Ansprüche 1, 2 oder 3 erhalten, trägt, dadurch gekennzeichnet, daß die Zusammensetzung der Pili durch wenigstens zwei Veränderungen der Proteinsequenz der Untereinheit oder Pilin modifiziert wurde, wobei diese Veränderungen aus dem Einfügen in das für die Untereinheit eines Pilus kodierenden Gens von wenigstens zwei für ein heterologes Peptid oder ein heterologes Protein kodierenden DMS-Fragmenten hervorgehen, die einen antigenen Bereich des Influenzavirus und/oder des Hamagglutinins des Influenzavirus bilden und/oder ein Peptid des Somatostatinhormons sind.

**5.** Verwendung von Proteinen, erhalten durch wenigstens zwei Modifikationen der Proteinsequenz der Untereinheit von Pili oder Pilin, die gemäß einem der Ansprüche 1, 2 oder 3 erhalten wurden, als Produkte für die tierische Gesundheit oder die menschliche Gesundheit.

**6.** Verwendung von Pili, Pilinen erhalten nach einem der Ansprüche 1, 2 oder 3 oder von Mikroorganismen, erhalten nach Anspruch 4, zum Erhalt eines pharmazeutischen Produkts, das zur medizinischen und/oder prophylaktischen Behandlung der menschlichen oder tierischen Gesundheit bestimmt ist.

**7.** Verwendung von Pili, Pilinen, erhalten nach einem der Ansprüche 1, 2 oder 3 oder von Mikroorganismen, erhalten nach Anspruch 4, für die Herstellung eines Mittels, das für die Durchführung von immunologischen Tests bestimmt ist.

38

## Fig. 1a

R 1 2 3 4 5 6 7 8 9 10

## Fig. 1b

1 2 3 4 5 6 7 8 9 10

# Fig. 2

# Fig. 3a

PBR322           K88AC

ataaactaccgcattaaagctAAACGGAGCCGCAGGGGATAGTTTTACGGTAATTCCGGAAAAATAAGGGGTTACCGATT

P1                                               BSTEII

TCAGTTTATTATTTGTGGAATATCAAGGGGGTTTATTTT

RBS

-21
MET LYS LYS THR LEU ILE ALA LEU ALA ILE ALA ALA SER ALA ALA SER GLY MET ALA HIS
ATG AAA AAG ACT CTG ATT GCA CTG GCA ATT GCT GCA TCT GCT GCA TCT GGT ATG GCA CAT
1
ALA TRP MET THR GLY ASP PHE ASN GLY SER VAL ASP ILE GLY GLY SER ILE THR ALA ASP
GCC TGG ATG ACT GGT GAT TTC AAT GGT TCG GTC GAT ATC GGT GGT AGT ATC ACT GCA GAT

                                 ECORV               PSTI

20
ASP TYR ARG GLN LYS TRP GLU TRP LYS VAL GLY THR GLY LEU ASN GLY PHE GLY ASN VAL
GAT TAT CGT CAG AAA TGG GAA TGG AAA GTT GGT ACA GGT CTT AAT GGA TTT GGT AAT GTA
40
LEU ASN ASP LEU THR ASN GLY GLY THR LYS LEU THR ILE THR VAL THR GLY ASN LYS PRO
TTG AAT GAC CTG ACC AAT GGT GGA ACC AAA CTG ACC ATT ACT GTT ACT GGT AAT AAG CCA
60
ILE LEU LEU GLY ARG THR LYS GLU ALA PHE ALA THR PRO VAL THR GLY GLY VAL ASP GLY
ATT TTG TTA GGC CGA ACC AAA GAA GCA TTT GCT ACG CCA GTA ACT GGT GGT GTA GAT GGA

                                                      ECO

80
ILE PRO HIS ILE ALA PHE THR ASP TYR GLU GLY ALA SER VAL VAL LEU ARG ASN PRO ASP
ATT CCT CAT ATT GCA TTT ACT GAC TAT GAA GGA GCT TCT GTA GTA CTC AGA AAC CCT GAT

RI                                            SCAI
100
GLY GLU THR ASN LYS LYS GLY LEU ALA TYR PHE VAL LEU PRO MET LYS ASN ALA GLU GLY
GGT GAA ACT AAT AAA AAA GGI TTA GCA TAT TTT GTT CTG CCG ATG AAA AAT GCA GAG GGC

# Fig. 3b

120
THR LYS VAL GLY SER VAL LYS VAL ASN ALA SER TYR ALA GLY VAL LEU GLY ARG GLY GLY
ACT AAA GTT GGT TCA GTG AAA GTG AAT GCA TCT TAT GCC GGT GTG TTA GGG AGA GGT GGG

NSII

140
VAL THR SER ALA ASP GLY GLU LEU LEU SER LEU PHE ALA ASP GLY LEU SER SER ILE PHE
GTT ACT TCT GCG GAC GGG GAG CTG CTT TCG CTT TTT GCC GAC GGG TTG AGC TCT ATC TTT

SACI

160
TYR GLY GLY LEU PRO ARG GLY SER GLU LEU SER ALA GLY SER ALA ALA ALA ALA ARG THR
TAT GGT GGT TTG CCG AGG GGT TCT GAA CTC TCG GCT GGG AGT GCC GCA GCG GCG CGC ACA

BGLI        BSSHII

180
LYS LEU PHE GLY SER LEU SER ARG ASN ASP ILE LEU GLY GLN ILE GLN ARG VAL ASN ALA
AAG TTG TTT GGA AGT CTA TCA AGA AAT GAT ATT CTC GGA CAG ATT CAA AGA GTA AAC GCA
200
ASN ILE THR SER LEU VAL ASP VAL ALA GLY SER TYR ARG GLU ASN MET GLU TYR THR ASP
AAT ATT ACT TCT CTT GTT GAC GTC GCA GGT TCT TAC AGG GAA AAC ATG GAG TAC ACT GAT

HINDII   AATII

220
GLY THR VAL VAL SER ALA ALA TYR ALA LEU GLY ILE ALA ASN GLY GLN THR ILE GLU ALA
GGA ACT GTT GTT TCT GCT GCC TAT GCA CTG GGT ATT GCA AAC GGT CAG ACT ATT GAG GCA
240
THR PHE ASN GLN ALA VAL THR THR SER THR GLN TRP SER ALA PRO LEU ASN VAL ALA ILE
ACT TTT AAT CAG GCT GTA ACT ACC AGC ACT CAG TGG AGC GCT CCG CTG AAC GTA GCA ATT
260
THR TYR TYR ***
ACT TAT TAC TAAGTTGTCGTGATGAGCTGCCAATTTATTATTGATACGTTCTGATAACAGACCAGCATCTTGGTG

TGGACGCTCTTTt

42

## Fig. 4

# Fig. 5a

1
TRP MET THR GLY ASP PHE ASN GLY SER VAL ASP ILE GLY GLY SER ILE THR ALA ASP
TGG ATG ACT GGT GAT TTC AAT GGT TCG GTC GAT ATC GGT GGT AGT ATC ACT GCA GAT
                            ─────          ───              ─────────
                            ECORV                           PSTI

20
ASP TYR ARG GLN LYS TRP GLU TRP GLY VAL GLY THR GLY LEU ASN GLU PHE GLY SER VAL
GAT TAT CGT CAG AAA TGG GAA TGG GGA GTT GGT ACA GGT CTT AAT GAA TTT GGT AGT GTA

40
LEU ASN ASP LEU THR ASN GLY GLY THR LYS LEU THR ILE THR VAL THR GLY ASN LYS PRO
TTG AAT GAC CTG ACC AAT GGT GGA ACC AAA CTG ACC ATT ACT GTT ACT GGT AAT AAG CCA

60
ILE LEU LEU GLY ARG THR LYS GLU ALA PHE ALA THR PRO VAL ALA SER GLY VAL ASP GLY
ATT TTG TTA GGC CGA ACC AAA GAA GCA TTT GCT ACG CCA GTG GCT AGT GGT GTA GAT GGA
                                                                           ───
                                                                           ECO

80
ILE PRO HIS ILE ALA PHE THR ASP TYR GLU GLY ALA SER VAL GLU LEU ARG ASN PRO ASP
ATT CCT CAT ATT GCA TTT ACT GAC TAT GAA GGA GCT TCT GTA GAA CTC AGA AAC CCT GAT
─── ─
RI

100
GLY GLU THR GLU LYS GLY LEU ALA TYR PHE VAL LEU PRO MET LYS ASN ALA GLU GLY THR
GGT GAA ACT GAA AAA GGT TTA GCA TAT TTT GTT CTG CCG ATG AAA AAT GCA GAG GGC ACT

120
LYS VAL GLY SER VAL LYS VAL ASN ALA SER TYR ALA GLY ALA LEU GLY ARG GLY GLY VAL
AAA GTT GGT TCA GTG AAA GTG AAT GCA TCT TAT GCC GGT GCG CTC GGG AGA GGT GGG GTT
                ─────────                               ───────
                NSII                                    AVAI

44

# Fig. 5b

```
140
THR SER ALA ASN GLY GLU LEU MET SER LEU PHE ALA GLU GLY SER HIS ALA ILE PHE TYR
ACT TCT GCG AAC GGG GAG CTG ATG TCG CTT TTT GCC GAA GGG TCG CAC GCT ATC TTT TAT
160
GLY GLY LEU PRO THR ASN VAL LYS ASN SER GLU LEU LYS GLY GLY SER ALA ALA ALA ALA
GGT GGT TTG CCG ACG AAT GTT AAG AAT TCT GAA CTC AAG GGT GGG AGT GCC GCA GCG GCG
                            ─────────                           ───────────── ═══
                              ECORI                                BGLI      BSS
180
ARG THR GLU LEU PHE GLY SER LEU SER LYS ASN ASP ILE LEU GLY GLN ILE GLN ARG VAL
CGC ACA GAG TTG TTT GGA AGT CTA TCA AAA AAT GAT ATT CTC GGA CAG ATT CAA AGA GTA
───
HII
200
ASN ALA ASN ILE THR SER LEU VAL ASN VAL PRO SER SER PHE ASN GLU ASN MET ALA TYR
AAC GCA AAT ATT ACT TCT CTT GTT AAC GTC CCA AGT TCT TTC AAT GAA AAC ATG GCG TAC
                        ─────────
                        HINDII/HPAI
220
THR ASP GLY SER VAL VAL SER VAL ALA TYR ALA LEU GLY ILE ALA ASN GLY GLN THR ILE
ACT GAT GGA TCT GTT GTT TCC GTT GCC TAT GCA CTG GGT ATT GCA AAC GGT CAG ACT ATT
            ─────────
            XHOII
240
GLU ALA THR PHE ASN GLN ALA VAL THR THR SER THR GLN TRP SER ALA PRO LEU ASN VAL
GAG GCA ACT TTT AAT CAG GCT GTA ACT ACC AGC ACT CAG TGG AGC GCT CCG CTG AAC GTA
260
ALA ILE THR TYR TYR ***
GCA ATT ACT TAT TAC TAA
```

## Fig. 6

Fig. 7

Fig. 8

# Fig. 9a

```
              10         20         30         40
ADUS   WHTGDFNGSV DIGGSITADD YRQKWEWGVG TGLNEFGSVL
ADNL                            K          G
AB1-                            K          G   N
AB2-                            K          G   N
ACUS                            K          G   N
ACNL                            K          G   N

. . . . . . .   . . . . . . . . . .   . . . . . . . . . .   . . . . . . . . . .   . . . . . . . . . .
              50         60         70         80
ADUS   NDLINGGIKL TITVTGNKPI LLGRTKEAFA TPVASGVDGI
ADNL        E          S          R          VG
AB1-                                         SG
AB2-                                         SG
ACUS                                         TG
ACNL                                         TG

. . . . . . .   . . . . . . . . . .   . . . . . . . . . .   . . . . . . . . . .   . . . . . . . . . .
              90        100      *  109        119
ADUS   PHIAFTDYEG ASVELRNPDG ETEK-GLAYF VLPMKNAEGI
ADNL                            I
AB1-    Q          K   T        N
AB2-    Q          K   T        N
ACUS               V            N K
ACNL               V            N K

. . . . . . .   . . . . . . . . . .   . . . . . . . . . .   . . . . . . . . . .   . . . . . . . . . .
              129        -139       149        159
ADUS   KVGSVKVNAS YAGALGRGGV TSANGELMSL FAEGSHAIFY
ADNL                            D
AB1-               VE K         D    F      D LR
AB2-               F K          D    F      D LR
ACUS               V            D    L      D LSS
ACNL               V            D    L      D LS

. . . . . . .   . . . . . . . . . .   . . . . . . . . . .   . . . . . . . . . .   . . . . . . . . . .
```

49

# Fig. 9b

```
          ***169          179          189          199
ADUS   GGLPTNVKNS ELKGGSAAAA RTELFGSLSK NDILGQIQRV
ADNL          Q   A PR
AB1-      I I SGA A TS                  R
AB2-      I   SG A TS                   R
ACUS       ---RG   SA        K          R
ACNL       ---RG   SSA       K          R

............ .......... .......... ..........
              209          219          229          239
ADUS   NANITSLVNV PSSFNENMAY TDGSVVSVAY ALGIANGQTI
ADNL              G                                R
AB1-           D  AG YR D E      T  A
AB2-           D  AG YR D E      T  A
ACUS           D  AG YR   E      T  A
ACNL           D  AG YR   E      T  A

...... .......... .......... .......... ..........
              249          259   264
ADUS   EATFNQAVTI STQWSAPLNV AITYY
ADNL
AB1-
AB2-
ACUS
ACNL
```

## Fig. 10a

AB1.PEP

## Fig. 10b

ADUS.PEP

# Fig. 11a

(1) pIX120 (262 AA)
-----------------

```
*   *   *                       *   *   *   *   *   *   *   *
L   S   S   I   F   Y   G   G   L   P   R   G   S   E   L   S   A
G AGC TCT ATC TTT TAT GGT GGT TTG CCG AGG GGT TCT GAA CTC TCG GCT
---------SACI

                                G   S   A   A   A   A   R
                                GGG AGT GCC GCA GCG GCG CGC
                                              BSSHII-------
```

(2) pIX126 (255)
-----------------

```
L   S   S   I   S   H   A   V   P   L   E   E   A   W   D   P   R
G AGC TCG ATA TCG CAT GCG GTA CCT CTA GAA GAA GCT TGG GAT CCG CGC

  SACI  ECORV  SPHI   KPNI   XBAI    HINDIII BAMHI  (BSSHII)
```

(3) pIX130 (265 AA)
-----------------

```
L   S   S   I   S   H   A   V   P   E   K   Q   T   R   G   I   F
G AGC TCG ATA TCG CAT GCG GTA CCG GAA AAA CAG ACC CGT GGT ATC TTC

  SACI  ECORV  SPHI   KPNI
                      G   A   L   E   E   A   W   D   P   R
                      GGT GCT CTA GAA GAA GCT TGG GAT CCG CGC

                          XBAI        HINDIII BAMHI
```

(4) pIX131 (260 AA)
-----------------

```
L   S   S   V   P   E   K   Q   T   R   G   I   F   G   A   L   E
G AGC TCG GTA CCG GAA AAA CAG ACC CGT GGT ATC TTC GGT GCT CTA GAA

SACI  KPNI                                        XBAI
                                      E   A   W   D   P   R
                                      GAA GCT TGG GAT CCG CGC

                                          HINDIII BAMHI
```

EP 0 264 150 B1

# Fig. 11b

```
(5) pIX123 (258 AA)
-------------------

L   S   S   I   F   Y   G   G   L   V   P   L   E   G   S   A   A
G AGC TCT ATC TTT TAT GGT GGT TTG GTA CCT CTA GAA GGG AGT GCC GCA
-------SACI                KPNI---------------XBAI
                                                    A   A   R
                                                    GCG GCG CGC
                                                    BSSHII------

(6) pIX136 (268 AA)
-------------------

L   S   S   I   F   Y   G   G   L   V   P   E   K   Q   T   R   G
G AGC TCT ATC TTT TAT GGT GGT TTG GTA CCG GAA AAA CAG ACC CGT GGT
-------SACI                    -------KPNI
                I   F   G   A   L   E   G   S   A   A   A   A   R
                ATC TTC GGT GCT CTA GAA GGG AGT GCC GCA GCG GCG CGC
                        XBAI------                       BSSHII------


(7) pIX139 (262 AA)
-------------------

L   S   S   V   P   E   K   Q - T   R   G   I   F   G   A   L   E
G AGC TCG GTA CCG GAA AAA CAG ACC CGT GGT ATC TTC GGT GCT CTA GAA
---------------                                          --------
SACI    KPNI                                             XBAI
                                    G   S   A   A   A   R
                                    GGG AGT GCC GCA GCG GCG CGC
                                                    BSSHII------
```

54

## Fig. 12

## Fig. 13a

## Fig. 13b

## Fig. 13c

## Fig. 13d

## Fig. 13e

# Fig. 13f

# Fig. 14

(1) pAD120 (264 AA)
----------------------

```
        *        *    *       *    *        *         *                        *
   V    N    V    P    S    S    E    N    E    N    M    A    Y    T    D    G    S
  GTT  AAC  GTC  CCA  AGT  TCT  TTC  AAT  GAA  AAC  ATG  GCG  TAC  ACT  GAT  GGA  TCT
  -------HINDII                                                    XHOII-------
```

(2) pAD121 (258 AA)
----------------------

```
   V    D    V    V    P    S    F    L    E    G    S
  GTT  GAC  GTG  GTA  CCA  AGC  TTT  CTA  GAG  GGA  TCT
  --------    -------------------------    ---------
  HINDII      KPNI     HINDIII XBAI        XHOII
```

(3) pAD122 (266 AA)
----------------------

```
   V    D    V    V    P    E    K    Q    T    R    G    I    F    G    A    L    E    G    S
  GTT  GAC  GTG  GTA  CCG  GAA  AAA  CAG  ACC  CGT  GGT  ATC  TTC  GGT  GCT  CTA  GAG  GGA  TCT
  -------    ------                          -                         -------    -------
  HINDII     KPNI                                                      XBAI       XHOII
```

## Fig. 15

a          b

## Fig. 16

## Fig. 17

# FIGURE 18

# FIGURE 19

# FIGURE 20

# FIGURE 21